# EUROPEAN PATENT APPLICATION

(11) **EP 4 620 444 A1**
(43) Date of publication of application: **24.09.2025**
(21) Application number: 23906734.1
(22) Date of filing: 06.12.2023
(51) Int. Cl.: A61F 13/53, A61F 13/532, A61F 13/534

(54) **ABSORBER AND ABSORBENT ARTICLE**

(30) Priority: 20.12.2022 JP 2022203113
(71) Applicant: Sumitomo Seika Chemicals Co., Ltd., Kako-gun, Hyogo 675-0145 (JP)
(72) Inventor: TAKAMATSU, Kazuyoshi, Himeji-shi, Hyogo 672-8076 (JP); KITANAKA, Hironobu, Himeji-shi, Hyogo 672-8076 (JP)
(74) Representative: Vossius & Partner Patentanwälte Rechtsanwälte mbB
(86) International application number: PCT/JP2023/043702
(87) International publication number: WO 2024/135368

(57) **Abstract**

An absorber including a water absorption layer including water-absorbent resin particles, in which the water absorption layer has a water absorption layer A and a water absorption layer B, the water absorption layer B has a strip shape, the water absorption layers A are disposed on both sides of the water absorption layer B in a transverse direction, the water absorption layer A has a 10-second value of swelling power of less than 5 N, and in a case where a 1-minute value of a lock-up height of the water absorption layer A is H_{A} and a 1-minute value of a lock-up height of the water absorption layer B is H_{B}, H_{A} - H_{B} is 1.5 cm/g or more.

## Description

### Technical Field

The present invention relates to an absorber, water-absorbent resin particles, and an absorbent article.

### Background Art

In order to absorb a liquid having water as a main component, such as urine, and to prevent the liquid from leaking, a water-absorbent article provided with an absorber that can absorb a liquid is known.

For example, Patent Literature 1 describes an absorbent article having a length along a longitudinal direction and a width along a width direction perpendicular to the longitudinal direction, in which the absorbent article is provided with an absorber extending along the longitudinal direction, a liquid permeable skin-facing sheet disposed on the skin-facing side of the absorber, a liquid impermeable non-skin-facing sheet disposed on the non-skin-facing side of the absorber, and a pair of SAP high-density regions which are formed of a group of particles of SAP as a superabsorbent polymer disposed in recesses on the surface of the absorber, and are disposed at both ends in at least one of the width direction or the longitudinal direction.

### Citation List

### Patent Literature

[Patent Literature 1] Japanese Unexamined Patent Publication No. 2022-106039

### Summary of Invention

### Technical Problem

According to the findings of the present inventors, in general, the permeation rate and the absorption capacity of an absorbent article are likely to be in a trade-off relationship. Specifically, in a case of attempting to increase the permeation rate of the absorbent article, it is effective to adopt a configuration in which a gel blocking phenomenon (a phenomenon in which a gap between particles is filled by expansion due to water absorption of water-absorbent resin particles) can be suppressed from occurring in the water-absorbent resin particles (for example, water-absorbent resin particles having a low water absorption rate). However, in a case where a liquid is continuously injected into the absorbent article, when trying to suppress the occurrence of the gel blocking phenomenon, the liquid tends to leak from an end part of the absorbent article without being completely absorbed (that is, the absorption capacity tends to decrease). On the other hand, in a case where the absorption capacity of the absorbent article is improved to suppress the leakage, it is effective to adopt a configuration in which the liquid hardly reaches the end part of the absorbent article even with the liquid being continuously injected (for example, water-absorbent resin particles having a high water absorption rate in which a gel blocking phenomenon is likely to occur). However, in this case, the permeation rate of the absorbent article tends to decrease due to the occurrence of the gel blocking phenomenon.

One aspect of the present invention relates to an absorber that can constitute an absorbent article having a high permeation rate and a large absorption capacity.

### Solution to Problem

The present disclosure includes the following items [1] to [9].
[1] An absorber includeing:
   a water absorption layer including water-absorbent resin particles,
   in which the water absorption layer has a water absorption layer A and a water absorption layer B,
   the water absorption layer B has a strip shape and the water absorption layers A are disposed on both sides of the water absorption layer B in a transverse direction,
   the water absorption layer A has a 10-second value of swelling power of less than 5 N, and
   in a case where a 1-minute value of a lock-up height of the water absorption layer A is H_{A} and a 1-minute value of a lock-up height of the water absorption layer B is H_{B}, H_{A} - H_{B} is 1.5 cm/g or more, a 10-second value of the swelling power is a value measured by a method including:
      spraying 0.1 g of the water-absorbent resin particles taken out from the water absorption layer over a mesh blocking one opening portion of a cylinder having an inner diameter of 20 mm to form a particle layer,
      disposing a cylindrical jig having a diameter of 19.5 mm and a mass of 20.5 g on the particle layer,
      disposing a water impermeable sheet on a horizontal surface of a sheet shaped glass filter permeated with physiological saline at 25°C,
      standing the cylinder, in which the particle layer and the cylindrical jig are placed, vertically on the water impermeable sheet in a direction through which the mesh is positioned at a lower position,
      removing the water impermeable sheet to allow the particle layer to absorb the physiological saline that has passed through the mesh, and
      measuring a force with which the swollen particle layer pushes up the cylindrical jig with a load cell at a time point when 10 seconds have elapsed after removing the water impermeable sheet, and recording the measured value as the 10-second value of the swelling power, and
      a 1-minute value of a lock-up height is a value measured by a method including:
         spraying 0.200 g of the water-absorbent resin particles taken out from the water absorption layer over an entire bottom surface of a cylinder having an inner diameter of 2.0 cm and a depth of 8.0 cm to form a particle layer having a height H0 [cm],
         injecting 20 g of physiological saline at 25°C into the particle layer to swell the particle layer,
         recording a maximum height of the particle layer at a time point when 1 minute has elapsed after the injection of a total amount of the physiological saline as H1, and
         calculating the 1-minute value of the lock-up height by an expression: (H1 - HO)/(Amount of water-absorbent resin particles).
[2] The absorber according to [1],
   in which an area of the water absorption layer B is 30% or less with respect to a total area of the water absorption layer.
[3] The absorber according to [1] or [2],
   in which an area of the water absorption layer B is less than 20% of a total area of the water absorption layer.
[4] The absorber according to any one of [1] to [3],
   in which the absorber has a strip shape, and
   the water absorption layer B is disposed such that a longitudinal direction of the water absorption layer B is along a longitudinal direction of the absorber.
[5] The absorber according to any one of [1] to [4],
   in which the water absorption layer includes a plurality of the water absorption layers B.
[6] Water-absorbent resin particles including:
   water-absorbent resin particles x having a 1-minute value of a lock-up height of less than 9 cm/g; and
   water-absorbent resin particles y having a 1-minute value of a lock-up height of 9 cm/g or more.
[7] The water-absorbent resin particles according to [6],
   in which the water-absorbent resin particles x includes polymer particles and a coating layer covering at least a part of a surface of the polymer particles.
[8] An absorber including:
   the water-absorbent resin particles according to [6] or [7].
[9] An absorbent article including:
   the absorber according to any one of [1] to [5] and [8].

### Advantageous Effects of Invention

According to one aspect of the present disclosure, it is possible to provide an absorber that can constitute an absorbent article having a high permeation rate and a large absorption capacity.

### Brief Description of Drawings

Fig. 1 is a schematic cross-sectional view of an absorber according to an embodiment.
Fig. 2 is a top view showing an example of the water absorption layer of the absorber shown in Fig. 1.
Fig. 3 is a top view showing an example of the water absorption layer of the absorber shown in Fig. 1.
Fig. 4 is a top view showing an example of the water absorption layer of the absorber shown in Fig. 1.
Fig. 5 is a top view showing an example of the water absorption layer of the absorber shown in Fig. 1.
Fig. 6 is a top view showing an example of the water absorption layer of the absorber shown in Fig. 1.
Fig. 7 is a plan view showing an example of an application pattern of an adhesive formed on a core wrap sheet.
Fig. 8 is a schematic cross-sectional view of an absorbent article according to an embodiment.
Fig. 9 is a schematic view showing a measurement device for a swelling power.
Fig. 10 is a schematic view showing a measurement device for a 45-degree leakage test.
Fig. 11 is a graph showing the results of Examples and Comparative Examples.

### Description of Embodiments

Hereinafter, several embodiments of the present invention will be described in detail. However, the present invention is not limited to the following embodiments.

In the present specification, "acryl" and "methacryl" collectively denote "(meth)acryl". "Acrylate" and "methacrylate" also similarly denote "(meth)acrylate". In the numerical value ranges described in a stepwise manner in the present specification, an upper limit or a lower limit of a numerical value range in a certain step can be optionally combined with an upper limit or a lower limit of a numerical value range in another step. In the numerical ranges described in the present specification, the upper limit or lower limit of the numerical ranges may be replaced with the values shown in the Examples. The materials exemplified in the present specification may be used alone or in combination of two or more kinds thereof. In a case where there are a plurality of substances corresponding to each component present in the composition, the content of each component in the composition means the total amount of the plurality of substances present in the composition unless otherwise specified. "Room temperature" means 25 ± 2°C. In addition to shape structures formed over the entire surface, the term "layer" also encompasses shape structures which are partially formed when observed in a plan view. "Physiological saline" denotes a 0.9 mass% sodium chloride aqueous solution.

### [Absorber]

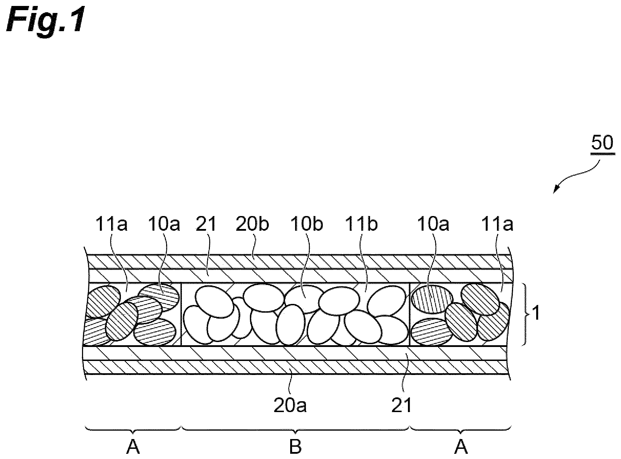

The absorber according to an embodiment includes a water absorption layer including water-absorbent resin particles. Fig. 1 is a schematic cross-sectional view showing an example of the absorber. An absorber 50 shown in Fig. 1 has a water absorption layer 1 and two core wrap sheets 20a and 20b. The core wrap sheets 20a and 20b are disposed at both sides of the water absorption layer 1. In other words, the water absorption layer 1 is disposed on the inner side of the core wrap sheets 20a and 20b. The shape of the water absorption layer 1 is retained by being sandwiched between the two core wrap sheets 20a and 20b. The core wrap sheets 20a and 20b may be two sheets, may be one folded sheet, or may be one bag body.

The water absorption layer 1 includes at least a water absorption layer A and a water absorption layer B. The water absorption layer A includes water-absorbent resin particles 10a and the water absorption layer B includes water-absorbent resin particles 10b. The water-absorbent resin particles 10a and 10b may be composed of only one kind of water-absorbent resin particles, or may be a mixture of two or more kinds of water-absorbent resin particles having different water-absorbent characteristics. Since the water absorption layer A and the water absorption layer B have different 1-minute values of a lock-up height as described later, the water-absorbent resin particles 10a of the water absorption layer A and the water-absorbent resin particles b of the water absorption layer B are water-absorbent resin particles different from each other. The water-absorbent resin particles 10b may be composed of only water-absorbent resin particles other than the water-absorbent resin particles 10a or may include the water-absorbent resin particles 10a in a part thereof. Similarly, the water-absorbent resin particles 10a may be composed of only water-absorbent resin particles other than the water-absorbent resin particles 10b or may include the water-absorbent resin particles 10b in a part thereof.

### (Water-Absorbent Resin Particles)

A shape of the water-absorbent resin particles is not particularly limited, and may be, for example, a substantially spherical shape, a crushed shape, or a granular shape, and particles obtained by aggregating the primary particles having these shapes may be formed.

A medium particle diameter of the water-absorbent resin particles may be 100 to 800 µm, 150 to 700 µm, 200 to 600 µm, or 250 to 500 µm. The medium particle diameter can be measured by the following method. JIS standard sieves are combined in the following order from the top: a sieve having an opening of 600 µm, a sieve having an opening of 500 µm, a sieve having an opening of 425 µm, a sieve having an opening of 300 µm, a sieve having an opening of 250 µm, a sieve having an opening of 180 µm, a sieve having an opening of 150 µm, and a tray. 50 g of the water-absorbent resin particles is put in the topmost sieve among the combined sieves, and classification is performed using a Ro-tap shaker (manufactured by Iida-seisakusho Japan Corporation) according to JIS Z 8815 (1994). After the classification, the mass of the particles remaining on each sieve is calculated as a mass percentage with respect to the total amount to determine a particle size distribution. The relationship between the opening of the sieve and the integrated value of the mass percentage of the particles remaining on the sieve is plotted on logarithmic probability paper by integrating in the order from the one having the largest particle diameter on the sieve with respect to this particle size distribution. By connecting the plots on the probability paper with a straight line, a particle diameter corresponding to the cumulative mass percentage of 50% by mass is obtained as the medium particle diameter.

The water-absorbent resin particles include , for example, polymer particles. The polymer particles may contain, for example, a crosslinked polymer having an ethylenically unsaturated monomer as a monomer unit. The polymer particles can be produced, for example, by a method including a step of polymerizing a monomer including an ethylenically unsaturated monomer. Examples of a polymerization method include a reverse phase suspension polymerization method, an aqueous solution polymerization method, a bulk polymerization method, and a precipitation polymerization method.

The ethylenically unsaturated monomer may be a water-soluble ethylenically unsaturated monomer. The water-soluble ethylenically unsaturated monomer include (meth)acrylic acid and a salt thereof, 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where the ethylenically unsaturated monomer has an amino group, the amino group may be quaternized. The ethylenically unsaturated monomer may be used alone or in combination of two or more kinds thereof.

In a case where the ethylenically unsaturated monomer has an acid group, the acid group may be neutralized using an alkaline neutralizing agent before being used in the polymerization reaction. The degree of neutralization in the ethylenically unsaturated monomer due to the alkaline neutralizing agent may be, for example, 10% to 100% by mole, 50% to 90% by mole, or 60% to 80% by mole of the acid groups in the ethylenically unsaturated monomer.

The polymer particles may further have a monomeric unit derived from a monomer other than the above-mentioned ethylenically unsaturated monomer. The ratio of the monomeric units derived from an ethylenically unsaturated monomer may be 70% to 100% by mole with respect to a total amount of the monomeric units constituting the polymer particles. The ratio of the monomeric units derived from (meth)acrylic acid and a salt thereof may be 70% to 100% by mole with respect to the total amount of the monomeric units constituting the crosslinking polymer in the polymer particles.

The crosslinked polymer constituting the polymer particles may be crosslinked with an internal crosslinking agent. The internal crosslinking agent is usually added to the reaction solution during the polymerization reaction. In a case of using the internal crosslinking agent, the water-absorbent characteristics of the water-absorbent resin particles (the water retention capacity of physiological saline, the water absorption rate according to the Vortex method, and the like) are easily controlled.

The polymer particles may be crosslinked with a surface crosslinking agent. The polymer in the vicinity of the surface of the polymer particles is mainly crosslinked by the surface crosslinking agent. In a case of using the surface crosslinking agent, the water-absorbent characteristics of the water-absorbent resin particles (the water retention capacity of physiological saline, the water absorption rate according to the Vortex method, and the like) are easily controlled.

The polymer particles may be substantially formed of only the crosslinked polymer, but may further contain, for example, various additional components selected from a gel stabilizer, a metal chelating agent, a flowability improver (lubricant), and the like. The additional components can be disposed inside the polymer particles, on the surface of the polymer particles, or both thereof. The additional component may be a flowability improver (lubricant).

The flowability improver may include inorganic particles. Examples of the inorganic particles include silica particles such as amorphous silica. In a case where the polymer particles include inorganic particles, the content of the inorganic particles may be 0.05 parts by mass to 5.0 parts by mass, 0.05 parts by mass to 3.0 parts by mass, 0.05 parts by mass to 1.0 part by mass, 0.05 parts by mass to 0.7 parts by mass, 0.05 parts by mass to 0.5 parts by mass, 0.1 parts by mass to 5.0 parts by mass, 0.1 parts by mass to 3.0 parts by mass, 0.1 parts by mass to 1.0 part by mass, 0.1 parts by mass to 0.7 parts by mass, 0.1 parts by mass to 0.5 parts by mass, 0.2 parts by mass to 5.0 parts by mass, 0.2 parts by mass to 3.0 parts by mass, 0.2 parts by mass to 1.0 part by mass, 0.2 parts by mass to 0.7 parts by mass, 0.2 parts by mass to 0.5 parts by mass, 0.3 parts by mass to 5.0 parts by mass, 0.3 parts by mass to 3.0 parts by mass, 0.3 parts by mass to 1.0 part by mass, 0.3 parts by mass to 0.7 parts by mass, 0.3 parts by mass to 0.5 parts by mass, 0.4 parts by mass to 5.0 parts by mass, 0.4 parts by mass to 3.0 parts by mass, 0.4 parts by mass to 1.0 part by mass, 0.4 parts by mass to 0.7 parts by mass, or 0.4 parts by mass to 0.5 parts by mass with respect to 100 parts by a total mass of the crosslinking polymer.

The water-absorbent resin particles may be composed of substantially only polymer particles, but may have, for example, a coating layer covering at least a part of the surface of the polymer particles. By forming a coating layer covering at least a part of the surface of the polymer particles, the polymer particles are less likely to come into contact with the liquid and it is easier to obtain water-absorbent resin particles having a low 1-minute value of the lock-up height.

The coating layer may include, for example, a polymer of <1> or <2> described below, or may include both the polymers of <1> and <2>.
<1> A polymer (a homopolymer, a copolymer, or a mixture thereof) including an alkene as a monomeric unit.
<2> A polymer (a homopolymer, a copolymer, or a mixture thereof) including an alkylene oxide as a monomeric unit.

In the polymer including an alkene as a monomeric unit, the alkene may be unsubstituted or may have a substituent. In addition, the polymer may be a homopolymer or a copolymer. The copolymer including an alkene as a constitutional unit may be a copolymer including only two or more kinds of alkenes as monomeric units, or a copolymer including one kind of alkenes and monomers other than the alkenes as monomeric units, or may be a copolymer including two or more kinds of alkenes and monomers other than the alkenes as monomeric units.

Examples of the alkene (unsubstituted alkene) constituting the copolymer include at least one selected from ethylene, propylene, and butene. The unsubstituted alkene (unsubstituted alkene) may be ethylene and/or propylene, or may be ethylene.

The monomer other than the alkene (unsubstituted alkene), which constitutes the copolymer, may be a water-soluble ethylenically unsaturated monomer. The water-soluble ethylenically unsaturated monomer may be the same as the compound exemplified as the water-soluble ethylenically unsaturated monomer constituting the polymer particles, or may be (meth)acrylic acid and/or a salt thereof.

The copolymer containing an alkene (unsubstituted alkene) as a monomer unit may be a copolymer containing ethylene and a water-soluble ethylenically unsaturated monomer as a monomer unit, a copolymer containing ethylene, (meth)acrylic acid, and/or a salt thereof as a monomer unit, a copolymer containing ethylene and an acrylic acid salt as a monomer unit, a copolymer containing ethylene and sodium acrylate, potassium acrylate, or ammonium acrylate as a monomer unit (an ethylene-sodium acrylate copolymer, an ethylene-potassium acrylate copolymer, or an ethylene-ammonium acrylate copolymer), or an ethylene-sodium acrylate copolymer.

In the copolymer including ethylene and a water-soluble ethylenically unsaturated monomer as monomeric units, the higher the ratio of the monomeric units derived from ethylene, the easier it is to increase the hydrophobicity of the coating layer. Therefore, the hydrophobicity of the coating layer can be controlled by the ratio of the monomeric units derived from ethylene. In addition, since the coating layer is more likely to adhere to the polymer particles as the ratio of the monomeric units derived from the water-soluble ethylenically unsaturated monomer is larger, the coating properties with respect to the polymer particles can be controlled by the ratio of the monomeric units derived from the water-soluble ethylenically unsaturated monomer. The ratio of the monomeric units derived from ethylene in the copolymer including ethylene and the water-soluble ethylenically unsaturated monomer as monomeric units may be 72.0% to 98.0% by mole or 80.0% to 97.0% by mole. By adjusting the ratio, a 10-second value of the swelling power and a 1-minute value of the lock-up height can be controlled.

The polymer containing alkylene oxide as a monomer unit may be a polyalkylene oxide (homopolymer) containing only one kind of alkylene oxide as a monomer unit, polyalkylene oxide (copolymer) containing two or more kinds of alkylene oxides as a monomer unit, or a copolymer containing one or two or more kinds of alkylene oxides and a monomer other than alkylene oxide as a monomer unit.

The alkylene oxide may be, for example, ethylene oxide and/or propylene oxide.

The polymer containing alkylene oxide as a monomer unit may be a homopolymer (polyethylene glycol) containing ethylene oxide as a monomer unit, a copolymer containing ethylene oxide and propylene oxide as monomer units, or a combination thereof.

The number-average molecular weight of the polymers including an alkylene oxide as a monomeric unit may be, for example, 100 to 200,000 or 700 to 50,000. The solubility of the polymers in water can be adjusted depending on the degree of the number-average molecular weight, and the 10-second value of the swelling power and the 1-minute value of the lock-up height can be controlled.

In a case where the coating layer includes <1> the polymer including an alkene as a monomeric unit and <2> the polymer including an alkylene oxide as a monomeric unit, the 10-second value of the swelling power and the 1-minute value of the lock-up height can be controlled by adjusting the mass ratio between the both.

The total content of the coating layer may be, for example, 1 to 40 parts by mass with respect to 100 parts by mass of the polymer particles on which the coating layer is formed.

The coating layer can be formed on at least a part of the surface, for example, by bringing the polymer particles into contact with a coating agent material. The coating agent material is, for example, a component capable of forming the above-mentioned coating layer or a material for forming the component.

The coating layer can be formed, for example, by (1) a method using an eggplant flask, (2) a method using a sprayer, or (3) a method using various granulators.

### (1) Method Using Eggplant Flask

In the method using an eggplant flask, first, a coating agent is injected into the eggplant flask, and then polymer particles are injected into the eggplant flask. The eggplant flask is attached to an evaporator and heated while rotating, and the liquid medium contained in the coating agent is distilled off under reduced pressure conditions. In this manner, coated-resin particles in which the surface of the polymer particles is coated with the coating layer are obtained.

### (2) Method Using Sprayer

In the method using a sprayer, first, the polymer particles are added to a separable flask provided with a stirrer blade, and the polymer particles is stirred. The polymer particles wound up by being stirred with the stirrer blade are sprayed with a coating agent. It is possible to spray the coating agent using, for example, a two-fluid type nozzle. From the viewpoint that uniform coating can be expected, it is desirable for the coating agent to be sprayed in a mist form using an air stream of an inert gas such as nitrogen. Thereafter, the contents of the separable flask are taken out, heated by a hot air dryer, and then cooled to room temperature to obtain coated-resin particles.

### (3) Method Using Various Granulators

Examples of granulators used for producing coated-resin particles include a rolling granulator, a stirring granulator, and a fluidized bed granulator.

In a case where a rolling granulator is used, an inclined shallow circular container provided in the rolling granulator rotates in advance, and polymer particles are supplied to the circular container while an appropriate amount of the coating agent is added. In this manner, the rolling particles are partially aggregated and a coating layer is formed on the surface thereof by a solvent or a dispersion medium included in the coating agent. Furthermore, an addition step of adding the polymer particles and the coating agent may be performed a plurality of times as necessary.

In a case where a stirring granulator is used, the polymer particles are injected into a mixer provided in the stirring granulator and stirred to be mixed while the coating agent is added thereto. In this manner, the particles during stirring are partially aggregated and a coating layer is formed on the surface thereof by the liquid medium included in the coating agent. The addition step of adding the polymer particles and the coating agent may be performed a plurality of times as necessary. Furthermore, excessive aggregation of the polymer particles can be suppressed by controlling a shearing force of the mixer.

In a case where a fluidized bed granulator is used, first, the polymer particles are injected into a container, which is provided in the fluidized bed granulator and in which hot air can be sent out from a lower portion, and the polymer particles are preliminarily fluidized. Thereafter, in a case where the coating agent is sprayed from a nozzle provided in the container, the polymer particles during stirring are partially aggregated by the liquid medium included in the coating agent and a coating layer is formed on the surface thereof. The coating agent may be sprayed a plurality of times as necessary. The excessive aggregation of the polymer particles may be suppressed by adjusting the spraying amount and/or the spraying frequency of the coating agent. For example, a fluidized bed granulator FBD/SG (manufactured by Yenchen Machinery Co., Ltd.) can be used as the fluidized bed granulator.

The 1-minute value of the lock-up height of the water-absorbent resin particles may be, for example, 0 cm/g to 50 cm/g, 0 cm/g to 40 cm/g, 0 cm/g to 30 cm/g, 0 cm/g to 20 cm/g, 0 cm/g to 16 cm/g, 0 cm/g to 14 cm/g, 0 cm/g to 12 cm/g, 0 cm/g to 10 cm/g, 0 cm/g to 9 cm/g, 0 cm/g to 8 cm/g, 0 cm/g to 6 cm/g, 0 cm/g to 4 cm/g, 0 cm/g to 2 cm/g, 0.5 cm/g to 50 cm/g, 0.5 cm/g to 40 cm/g, 0.5 cm/g to 30 cm/g, 0.5 cm/g to 20 cm/g, 0.5 cm/g to 16 cm/g, 0.5 cm/g to 14 cm/g, 0.5 cm/g to 12 cm/g, 0.5 cm/g to 10 cm/g, 0.5 cm/g to 9 cm/g, 0.5 cm/g to 8 cm/g, 0.5 cm/g to 6 cm/g, 0.5 cm/g to 4 cm/g, 0.5 cm/g to 2 cm/g, 1 cm/g to 50 cm/g, 1 cm/g to 40 cm/g, 1 cm/g to 30 cm/g, 1 cm/g to 20 cm/g, 1 cm/g to 16 cm/g, 1 cm/g to 14 cm/g, 1 cm/g to 12 cm/g, 1 cm/g to 10 cm/g, 1 cm/g to 9 cm/g, 1 cm/g to 8 cm/g, 1 cm/g to 6 cm/g, 1 cm/g to 4 cm/g, 1 cm/g to 2 cm/g, 5 cm/g to 50 cm/g, 5 cm/g to 40 cm/g, 5 cm/g to 30 cm/g, 5 cm/g to 20 cm/g, 5 cm/g to 16 cm/g, 5 cm/g to 14 cm/g, 5 cm/g to 12 cm/g, 9 cm/g to 50 cm/g, 9 cm/g to 40 cm/g, 9 cm/g to 30 cm/g, 9 cm/g to 20 cm/g, 9 cm/g to 16 cm/g, 9 cm/g to 14 cm/g, 9 cm/g to 12 cm/g, 10 cm/g to 50 cm/g, 10 cm/g to 40 cm/g, 10 cm/g to 30 cm/g, 10 cm/g to 20 cm/g, 10 cm/g to 16 cm/g, 10 cm/g to 14 cm/g, or 10 cm/g to 12 cm/g.

The 1-minute value of the lock-up height of the water-absorbent resin particles is a value measured by a method including spraying 0.200 g of the water-absorbent resin particles over an entire bottom surface of a cylinder having an inner diameter of 2.0 cm and a depth of 8.0 cm to form a particle layer having a height H0 [cm], injecting 20 g of physiological saline into the particle layer to swell the particle layer, recording a maximum height of the particle layer at a time point when 1 minute has elapsed after the injection of a total amount of the physiological saline as H1 [cm], and calculating the 1-minute value of the lock-up height by an expression: (H1 - HO)/(Amount of water-absorbent resin particles). Furthermore, in a case where the water-absorbent resin particles include two or more kinds of water-absorbent resin particles at a predetermined ratio, 0.200 g of the water-absorbent resin particles sprayed in the cylinder also includes two or more kinds of water-absorbent resin particles at the predetermined ratio.

The water-absorbent resin particles may include water-absorbent resin particles x having a 1-minute value of a lock-up height of less than 9 cm/g and water-absorbent resin particles y having a 1-minute value of a lock-up height of 9 cm/g or more.

Since the water-absorbent resin particles includes the coating layer, the 1-minute value of the lock-up height is relatively small. Therefore, the water-absorbent resin particles x may include polymer particles and a coating layer covering at least a part of the surface of the polymer particles. The water-absorbent resin particles y may be polymer particles including a coating layer or polymer particles not including a coating layer.

The 1-minute value of the lock-up height of the water-absorbent resin particles x may be, for example, 0 cm/g or more and less than 9 cm/g, 0 cm/g to 8 cm/g, 0 cm/g to 7 cm/g, 0 cm/g to 6 cm/g, 0 cm/g to 5 cm/g, 0 cm/g to 4 cm/g, 0 cm/g to 3 cm/g, 0 cm/g to 2 cm/g, 0 cm/g to 1.5 cm/g, 0.5 cm/g or more and less than 9 cm/g, 0.5 cm/g to 8 cm/g, 0.5 cm/g to 7 cm/g, 0.5 cm/g to 6 cm/g, 0.5 cm/g to 5 cm/g, 0.5 cm/g to 4 cm/g, 0.5 cm/g to 3 cm/g, 0.5 cm/g to 2 cm/g, 0.5 cm/g to 1.5 cm/g, 1 cm/g or more and less than 9 cm/g, 1 cm/g to 8 cm/g, 1 cm/g to 7 cm/g, 1 cm/g to 6 cm/g, 1 cm/g to 5 cm/g, 1 cm/g to 4 cm/g, 1 cm/g to 3 cm/g, 1 cm/g to 2 cm/g, or 1 cm/g to 1.5 cm/g.

The 1-minute value of the lock-up height of the water-absorbent resin particles y may be, for example, 9 cm/g to 50 cm/g, 9 cm/g to 40 cm/g, 9 cm/g to 30 cm/g, 9 cm/g to 20 cm/g, 9 cm/g to 16 cm/g, 9 cm/g to 14 cm/g, 9 cm/g to 12 cm/g, 9 cm/g to 10 cm/g, 10 cm/g to 50 cm/g, 10 cm/g to 40 cm/g, 10 cm/g to 30 cm/g, 10 cm/g to 20 cm/g, 10 cm/g to 16 cm/g, 10 cm/g to 14 cm/g, 10 cm/g to 12 cm/g, 11 cm/g to 50 cm/g, 11 cm/g to 40 cm/g, 11 cm/g to 30 cm/g, 11 cm/g to 20 cm/g, 11 cm/g to 16 cm/g, 11 cm/g to 14 cm/g, 11 cm/g to 12 cm/g, 12 cm/g to 50 cm/g, 12 cm/g to 40 cm/g, 12 cm/g to 30 cm/g, 12 cm/g to 20 cm/g, 12 cm/g to 16 cm/g, 12 cm/g to 14 cm/g, 13 cm/g to 50 cm/g, 13 cm/g to 40 cm/g, 13 cm/g to 30 cm/g, 13 cm/g to 20 cm/g, 13 cm/g to 16 cm/g, 13 cm/g to 14 cm/g, 14 cm/g to 50 cm/g, 14 cm/g to 40 cm/g, 14 cm/g to 30 cm/g, 14 cm/g to 20 cm/g, or 14 cm/g to 16 cm/g.

In the water-absorbent resin particles, the ratio of the mass of the water-absorbent resin particles y to the mass of the water-absorbent resin particles x (Mass of water-absorbent resin particles y/Mass of water-absorbent resin particles x) may be 0.1 to 15, 0.1 to 12, 0.1 to 10, 0.1 to 8, 0.1 to 6, 0.1 to 4, 0.1 to 3, 0.1 to 2.5, 0.1 to 2, 0.1 to 1.5, 0.1 to 1, 0.25 to 15, 0.25 to 12, 0.25 to 10, 0.25 to 8, 0.25 to 6, 0.25 to 4, 0.25 to 3, 0.25 to 2.5, 0.25 to 2, 0.25 to 1.5, 0.25 to 1, 0.5 to 15, 0.5 to 12, 0.5 to 10, 0.5 to 8, 0.5 to 6, 0.5 to 4, 0.5 to 3, 0.5 to 2.5, 0.5 to 2, 0.5 to 1.5, 0.5 to 1, 1 to 15, 1 to 12, 1 to 10, 1 to 8, 1 to 6, 1 to 4, 1 to 3, 1 to 2.5, 1 to 2, 1 to 1.5, 1.5 to 15, 1.5 to 12, 1.5 to 10, 1.5 to 8, 1.5 to 6, 1.5 to 4, 1.5 to 3, 1.5 to 2.5, 1.5 to 2, 2 to 15, 2 to 12, 2 to 10, 2 to 8, 2 to 6, 2 to 4, 2 to 3, or 2 to 2.5. The smaller the ratio of the mass of the water-absorbent resin particles y to the mass of the water-absorbent resin particles x (Mass of water-absorbent resin particles y/Mass of water-absorbent resin particles x), the higher the permeation rate, and the larger the absorption capacity tends to be.

The 10-second value of the swelling power of the water-absorbent resin particles may be, for example, more than 0 N and less than 5 N, more than 0 N and 4.6 N or less, more than 0 N and 4.3 N or less, more than 0 N and 3.5 N or less, more than 0 N and 3 N or less, more than 0 N and 2.5 N or less, more than 0 N and 2.2 N or less, 0.5 N or more and less than 5 N, 0.5 N to 4.6 N, 0.5 N to 4.3 N, 0.5 N to 3.5 N, 0.5 N to 3 N, 0.5 N to 2.5 N, 0.5 N to 2.2 N, 1 N or more and less than 5 N, 1 N to 4.6 N, 1 N to 4.3 N, 1 N to 3.5 N, 1 N to 3 N, 1 N to 2.5 N, 1 N to 2.2 N, 1.5 N or more and less than 5 N, 1.5 N to 4.6 N, 1.5 N to 4.3 N, 1.5 N to 3.5 N, 1.5 N to 3 N, 1.5 N to 2.5 N, 1.5 N to 2.2 N, 2 N or more and less than 5 N, 2 N to 4.6 N, 2 N to 4.3 N, 2 N to 3.5 N, 2 N to 3 N, 2 N to 2.5 N, or 2 N to 2.2 N.

The 10-second value of the swelling power of the water-absorbent resin particles is a value measured by a method including spraying 0.1 g of the water-absorbent resin particles over a mesh blocking one opening portion of a cylinder having an inner diameter of 20 mm to form a particle layer, disposing a cylindrical jig having a diameter of 19.5 mm and a mass of 20.5 g on the particle layer, disposing a water impermeable sheet on a horizontal surface of a sheet shaped glass filter permeated with physiological saline at 25°C, standing the cylinder, in which the particle layer and the cylindrical jig are placed, vertically on the water impermeable sheet in a direction through which the mesh is positioned at a lower position, removing the water impermeable sheet to allow the particle layer to absorb the physiological saline that has passed through the mesh, measuring a force with which the swollen particle layer pushes up the cylindrical jig with a load cell at a time point when 10 seconds have elapsed after removing the water impermeable sheet, and recording the measured value as the 10-second value of the swelling power. Furthermore, in a case where the water-absorbent resin particles include two or more kinds of water-absorbent resin particles at a predetermined ratio, 0.1 g of the water-absorbent resin particles sprayed over the mesh also includes two or more kinds of water-absorbent resin particles at the predetermined ratio.

### (Characteristics of Water Absorption Layer)

The 10-second value of the swelling power of the water absorption layer A may be less than 5 N, and may be, for example, more than 0 N and less than 5 N, more than 0 N and 4.6 N or less, more than 0 N and 4.3 N or less, more than 0 N and 3.5 N or less, more than 0 N and 3 N or less, more than 0 N and 2.5 N or less, more than 0 N and 2.2 N or less, 0.5 N or more and less than 5 N, 0.5 N to 4.6 N, 0.5 N to 4.3 N, 0.5 N to 3.5 N, 0.5 N to 3 N, 0.5 N to 2.5 N, 0.5 N to 2.2 N, 1 N or more and less than 5 N, 1 N to 4.6 N, 1 N to 4.3 N, 1 N to 3.5 N, 1 N to 3 N, 1 N to 2.5 N, 1 N to 2.2 N, 1.5 N or more and less than 5 N, 1.5 N to 4.6 N, 1.5 N to 4.3 N, 1.5 N to 3.5 N, 1.5 N to 3 N, 1.5 N to 2.5 N, 1.5 N to 2.2 N, 2 N or more and less than 5 N, 2 N to 4.6 N, 2 N to 4.3 N, 2 N to 3.5 N, 2 N to 3 N, 2 N to 2.5 N, or 2 N to 2.2 N.

The 10-second value of the swelling power of the water absorption layer B may be, for example, more than 0 N and less than 4 N, more than 0 N and 3.6 N or less, more than 0 N and 3 N or less, more than 0 N and 2 N or less, more than 0 N and 1.3 N or less, more than 0 N and 1 N or less, 0.2 N or more and less than 4 N, 0.2 N to 3.6 N, 0.2 N to 3 N, 0.2 N to 2 N, 0.2 N to 1.3 N, 0.2 to 1 N, 0.5 N or more and less than 4 N, 0.5 N to 3.6 N, 0.5 N to 3 N, 0.5 N to 2 N, 0.5 N to 1.3 N, or 0.5 to 1 N.

The 10-second value of the swelling power of the water absorption layer A or the water absorption layer B is a value measured by a method including spraying 0.1 g of the water-absorbent resin particles a or the water-absorbent resin particles b included in the water absorption layer A or the water absorption layer B over a mesh blocking one opening portion of a cylinder having an inner diameter of 20 mm to form a particle layer, disposing a cylindrical jig having a diameter of 19.5 mm and a mass of 20.5 g on the particle layer, disposing a water impermeable sheet on a horizontal surface of a sheet shaped glass filter permeated with physiological saline at 25°C, vertically the cylinder, in which the particle layer and the cylindrical jig are placed, vertically on the water impermeable sheet in a direction through which the mesh is positioned at a lower position, removing the water impermeable sheet to allow the particle layer to absorb the physiological saline that has passed through the mesh, measuring a force with which the swollen particle layer pushes up the cylindrical jig with a load cell at a time point when 10 seconds have elapsed after removing the water impermeable sheet, and recording the measured value as the 10-second value of the swelling power. Furthermore, in a case where the water-absorbent resin particles a or the water-absorbent resin particles b include two or more kinds of water-absorbent resin particles at a predetermined ratio, 0.1 g of the water-absorbent resin particles a or the water-absorbent resin particles b sprayed over the mesh also includes two or more kinds of water-absorbent resin particles at the predetermined ratio.

The 1-minute value (H_{A}) of the lock-up height of the water absorption layer A may be, for example, 6 cm/g to 50 cm/g, 6 cm/g to 40 cm/g, 6 cm/g to 30 cm/g, 6 cm/g to 20 cm/g, 6 cm/g to 16 cm/g, 8 cm/g to 50 cm/g, 8 cm/g to 40 cm/g, 8 cm/g to 30 cm/g, 8 cm/g to 20 cm/g, 8 cm/g to 16 cm/g, 10 cm/g to 50 cm/g, 10 cm/g to 40 cm/g, 10 cm/g to 30 cm/g, 10 cm/g to 20 cm/g, 10 cm/g to 16 cm/g, 11 cm/g to 50 cm/g, 11 cm/g to 40 cm/g, 11 cm/g to 30 cm/g, 11 cm/g to 20 cm/g, 11 cm/g to 16 cm/g, 12 cm/g to 50 cm/g, 12 cm/g to 40 cm/g, 12 cm/g to 30 cm/g, 12 cm/g to 20 cm/g, 12 cm/g to 16 cm/g, 14 cm/g to 50 cm/g, 14 cm/g to 40 cm/g, 14 cm/g to 30 cm/g, 14 cm/g to 20 cm/g, or 14 cm/g to 16 cm/g.

The 1-minute value (H_{B}) of the lock-up height of the water absorption layer B may be, for example, 1 cm/g to 30 cm/g, 1 cm/g to 25 cm/g, 1 cm/g to 20 cm/g, 1 cm/g to 15 cm/g, 1 cm/g to 13.5 cm/g, 1.5 cm/g to 30 cm/g, 1.5 cm/g to 25 cm/g, 1.5 cm/g to 20 cm/g, 1.5 cm/g to 15 cm/g, 1.5 cm/g to 13.5 cm/g, 2 cm/g to 30 cm/g, 2 cm/g to 25 cm/g, 2 cm/g to 20 cm/g, 2 cm/g to 15 cm/g, 2 cm/g to 13.5 cm/g, 3 cm/g to 30 cm/g, 3 cm/g to 25 cm/g, 3 cm/g to 20 cm/g, 3 cm/g to 15 cm/g, 3 cm/g to 13.5 cm/g, 4 cm/g to 30 cm/g, 4 cm/g to 25 cm/g, 4 cm/g to 20 cm/g, 4 cm/g to 15 cm/g, 4 cm/g to 13.5 cm/g, 5 cm/g to 30 cm/g, 5 cm/g to 25 cm/g, 5 cm/g to 20 cm/g, 5 cm/g to 15 cm/g, or 5 cm/g to 13.5 cm/g.

A difference (H_{A} - H_{B}) between the 1-minute value (H_{A}) of the lock-up height of the water absorption layer A and the 1-minute value (H_{B}) of the lock-up height of the water absorption layer B is 1.5 cm/g or more. The difference may be, for example, 1.5 cm/g to 20 cm/g, 1.5 cm/g to 18 cm/g, 1.5 cm/g to 16 cm/g, 1.5 cm/g to 14 cm/g, 1.5 cm/g to 11 cm/g, 1.5 cm/g to 10 cm/g, 1.5 cm/g to 9 cm/g, 1.8 cm/g to 20 cm/g, 1.8 cm/g to 18 cm/g, 1.8 cm/g to 16 cm/g, 1.8 cm/g to 14 cm/g, 1.8 cm/g to 11 cm/g, 1.8 cm/g to 10 cm/g, 1.8 cm/g to 9 cm/g, 2 cm/g to 20 cm/g, 2 cm/g to 18 cm/g, 2 cm/g to 16 cm/g, 2 cm/g to 14 cm/g, 2 cm/g to 11 cm/g, 2 cm/g to 10 cm/g, 2 cm/g to 9 cm/g, 2.5 cm/g to 20 cm/g, 2.5 cm/g to 18 cm/g, 2.5 cm/g to 16 cm/g, 2.5 cm/g to 14 cm/g, 2.5 cm/g to 11 cm/g, 2.5 cm/g to 10 cm/g, 2.5 cm/g to 9 cm/g, 3 cm/g to 20 cm/g, 3 cm/g to 18 cm/g, 3 cm/g to 16 cm/g, 3 cm/g to 14 cm/g, 3 cm/g to 11 cm/g, 3 cm/g to 10 cm/g, 3 cm/g to 9 cm/g, 5 cm/g to 20 cm/g, 5 cm/g to 18 cm/g, 5 cm/g to 16 cm/g, 5 cm/g to 14 cm/g, 5 cm/g to 11 cm/g, 5 cm/g to 10 cm/g, 5 cm/g to 9 cm/g, 7 cm/g to 20 cm/g, 7 cm/g to 18 cm/g, 7 cm/g to 16 cm/g, 7 cm/g to 14 cm/g, 7 cm/g to 11 cm/g, 7 cm/g to 10 cm/g, or 7 cm/g to 9 cm/g.

The 1-minute value of the lock-up height of the water absorption layer A or the water absorption layer B is a value measured by a method including spraying 0.200 g of the water-absorbent resin particles a or the water-absorbent resin particles b include the water absorption layer A or the water absorption layer B over an entire bottom surface of a cylinder having an inner diameter of 2.0 cm and a depth of 8.0 cm to form a particle layer having a height H0 [cm], injecting 20 g of physiological saline to the particle layer to swell the particle layer, recording a maximum height of the particle layer at a time point when 1 minute has elapsed after the injection of a total amount of the physiological saline as H1 [cm], and calculating the 1-minute value of the lock-up height by an expression: (H1 - HO)/(Amount of water-absorbent resin particles). Furthermore, in a case where the water-absorbent resin particles a or the water-absorbent resin particles b include two or more kinds of water-absorbent resin particles at a predetermined ratio, 0.200 g of the water-absorbent resin particles a or the water-absorbent resin particles b sprayed in the cylinder also includes two or more kinds of water-absorbent resin particles at the predetermined ratio.

The 10-second value of the swelling power can be controlled, for example, by changing the shape of the water-absorbent resin particles to adjust the specific surface area, performing a surface treatment such as hydrophilizing or hydrophobizing the surface of the water-absorbent resin, and adjusting a balance of the intensity of the crosslinking density in one particle by internal crosslinking and surface crosslinking, and the 1-minute value of the lock-up height can be controlled by carrying out a surface treatment such as hydrophilizing or hydrophobizing the surface of the water-absorbent resin, and covering the polymer particles with the coating layer, or the like.

The water-absorbent resin particles 10a included in the water absorption layer A may include water-absorbent resin particles 10aa in which the water absorption amount of the physiological saline is, for example, 20 to 80 g/g, 25 to 70 g/g, or 30 to 60 g/g. The water absorption rate (Vortex method) of the water-absorbent resin particles 10aa may be, for example, 1 to 70 seconds, 5 to 60 seconds, 10 to 50 seconds, or 15 to 45 seconds. The 1-minute value of the lock-up height of the water-absorbent resin particles 10aa may be, for example, 10.7 cm/g to 20.0 cm/g, 10.7 cm/g to 18.0 cm/g, 10.7 cm/g to 17.0 cm/g, 10.7 cm/g to 16.0 cm/g, 11.0 cm/g to 20.0 cm/g, 11.0 cm/g to 18.0 cm/g, 11.0 cm/g to 17.0 cm/g, or 11.0 cm/g to 16.0 cm/g. The 10-second value of the swelling power of the water-absorbent resin particles 10aa may be, for example, 0.08 N to 5.5 N, 0.08 N to 5.0 N, 0.08 N to 4.6 N, 0.1 N to 5.5 N, 0.1 N to 5.0 N, or 0.1 N to 4.6 N.

The ratio of the water-absorbent resin particles 10aa in the entire water-absorbent resin particles 10a may be 80% by mass to 100% by mass, 90% by mass to 100% by mass, 95% by mass to 100% by mass, 98% by mass to 100% by mass, or 100% by mass. In a case where the water-absorbent resin particles 10a include water-absorbent resin particles 10ab other than the water-absorbent resin particles 10aa, the water-absorbent resin particles 10ab may be water-absorbent resin particles 10ba which will be described later.

The water-absorbent resin particles 10b included in the water absorption layer B may include water-absorbent resin particles 10ba in which the water absorption amount of the physiological saline is, for example, 20 to 80 g/g, 25 to 70 g/g, or 30 to 60 g/g. The water absorption rate (Vortex method) of the water-absorbent resin particles 10ba may be, for example, 10 to 1,000 seconds, 15 to 900 seconds, 20 seconds to 800 seconds, or 25 seconds to 700 seconds. The 1-minute value of the lock-up height of the water-absorbent resin particles 10ba may be, for example, more than 0 cm/g and 15.0 cm/g or less, more than 0 cm/g and 14.0 cm/g or less, more than 0 cm/g and 13.5 cm/g or less, 0.2 cm/g to 15.0 cm/g, 0.2 cm/g to 14.0 cm/g, 0.2 cm/g to 13.5 cm/g, 0.5 cm/g to 15.0 cm/g, 0.5 cm/g to 14.0 cm/g, or 0.5 cm/g to 13.5 cm/g. The 10-second value of the swelling power of the water-absorbent resin particles 10ba may be, for example, 0 N to 2.0 N, 0 N to 1.8 N, 0 N to 1.5 N, 0 N to 1.4 N, 0.2 N to 2.0 N, 0.2 N to 1.8 N, 0.2 N to 1.5 N, or 0.2 N to 1.4 N.

The ratio of the water-absorbent resin particles 10ba in the entire water-absorbent resin particles 10b may be 30% by mass to 100% by mass, 30% by mass to 90% by mass, 30% by mass to 80% by mass, 30% by mass to 70% by mass, 30% by mass to 60% by mass, 40% by mass to 100% by mass, 40% by mass to 90% by mass, 40% by mass to 80% by mass, 40% by mass to 70% by mass, 40% by mass to 60% by mass, 50% by mass to 100% by mass, 50% by mass to 90% by mass, 50% by mass to 80% by mass, 50% by mass to 70% by mass, or 50% by mass to 60% by mass. In a case where the water-absorbent resin particles 10b include water-absorbent resin particles 10bb other than the water-absorbent resin particles 10ba, the water-absorbent resin particles 10bb may be the above-mentioned water-absorbent resin particles 10aa.

The water absorption amount and the water absorption rate of the water-absorbent resin particles in physiological saline can be measured by methods described in Examples which will be described later.

A difference (W_{A} - W_{B}) between the mass (W_{A}) of the water-absorbent resin particles a per unit area of the water absorption layer A and the mass (W_{B}) of the water-absorbent resin particles b per unit area of the water absorption layer B may be, for example,-0.03 g/cm² to 1 g/cm², -0.03 g/cm² to 0.8 g/cm², -0.03 g/cm² to 0.5 g/cm², -0.03 g/cm² to 0.3 g/cm², -0.03 g/cm² to 0.1 g/cm², -0.03 g/cm² to 0.03 g/cm², -0.03 g/cm² to 0.01 g/cm², -0.01 g/cm² to 1 g/cm², -0.01 g/cm² to 0.8 g/cm², - 0.01 g/cm² to 0.5 g/cm², -0.01 g/cm² to 0.3 g/cm², -0.01 g/cm² to 0.1 g/cm², -0.01 g/cm² to 0.03 g/cm², -0.01 g/cm² to 0.01 g/cm², or 0 g/cm².

In the absorber according to an embodiment of the present invention, even in a case where W_{A} - W_{B} is in the above-mentioned range (that is, the difference in the amount of water-absorbent resin particles per unit area between the water absorption layer A and the water absorption layer B is extremely small), it is considered that the liquid can diffuse to the entire absorber along the water absorption layer B due to mechanism of action which will be described later since the difference (H_{A} - H_{B}) between the 1-minute value (H_{A}) of the lock-up height of the water absorption layer A and the 1-minute value (H_{B}) of the lock-up height of the water absorption layer B is 1.5 cm/g or more. Therefore, by using the absorber according to the embodiment of the present invention, it is possible to constitute an absorbent article having a high permeation rate and a large absorption capacity.

A difference (T_{A} - T_{B}) between the thickness of the water absorption layer A (T_{A}) and the thickness of the water absorption layer B (T_{B}) may be, for example, -0.4 mm to 0.4 mm, -0.4 mm to 0.3 mm, -0.4 mm to 0.2 mm, -0.4 mm to 0.1 mm, -0.3 mm to 0.4 mm, -0.3 mm to 0.3 mm, -0.3 mm to 0.2 mm, -0.3 mm to 0.1 mm, -0.2 mm to 0.4 mm, -0.2 mm to 0.3 mm, -0.2 mm to 0.2 mm, -0.2 mm to 0.1 mm, -0.1 mm to 0.4 mm, -0.1 mm to 0.3 mm, -0.1 mm to 0.2 mm, -0.1 mm to 0.1 mm, or 0 mm.

In the absorber according to the embodiment of the present invention, it is considered that even in a case where the difference (T_{A} - T_{B}) between the thickness of the water absorption layer A (T_{A}) and the thickness of the water absorption layer B (T_{B}) is small as listed above (that is, in a case where there is almost no level difference between the water absorption layer A and the water absorption layer B), the liquid can diffuse along the water absorption layer B to the entire absorber due to estimated mechanism which will be described later. Therefore, by using the absorber according to the embodiment of the present invention, it is possible to constitute an absorbent article having a high permeation rate and a large absorption capacity.

The content of the water-absorbent resin particles a or the water-absorbent resin particles b in the water absorption layer A or the water absorption layer B may be 90% by mass to 100% by mass, 95% by mass to 100% by mass, 98% by mass to 100% by mass, or 100% by mass with respect to the mass of each water absorption layer. The content of the water-absorbent resin particles a in the water absorption layer A and the content of the water-absorbent resin particles b in the water absorption layer B may be the same as or different from each other.

### (Disposition of Water Absorption Layer)

In a plan view of the absorber 50, the shapes of the water absorption layer 1, the water absorption layer A, and the water absorption layer B are not particularly limited, and may be, for example, a rectangular shape, a circular shape, or an elliptical shape.

In the plan view of the absorber 50, the water absorption layer A, the water absorption layer B, and the water absorption layer A are disposed in this order along at least one direction of the water absorption layer 1 (for example, the water absorption layer 1 in the transverse direction). The absorber may include a plurality of water absorption layers A and may include a plurality of water absorption layers B.

In the plan view of the absorber 50, the water absorption layer A and the water absorption layer B may or may not be adjacent to each other. In a case where the water absorption layer A and the water absorption layer B are not adjacent to each other, for example, the water absorption layer A, a fiber layer which will be described later, and the water absorption layer B may be disposed in this order.

In the plan view of the absorber 50, the water absorption layer B may be disposed to straddle the center line of the water absorption layer 1 in at least one direction. Specifically, the water absorption layer B may be disposed to straddle the center line of the water absorption layer 1 in the longitudinal direction (a line passing through the center of the water absorption layer 1 in the longitudinal direction and orthogonal to the longitudinal direction of the water absorption layer 1) and/or the center line of the water absorption layer 1 in the transverse direction (a line passing through the center of the water absorption layer 1 in the transverse direction and orthogonal to the water absorption layer 1 in the transverse direction).

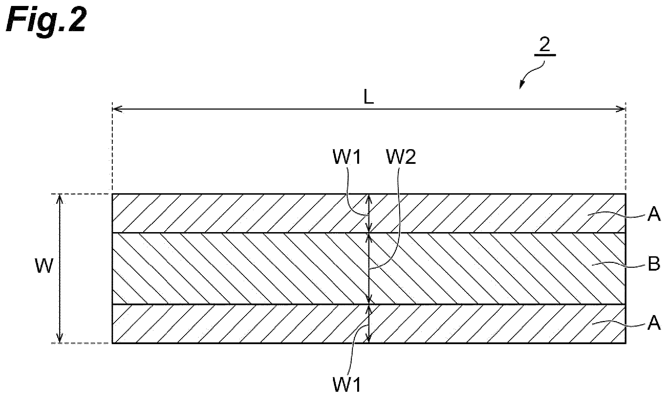

In the plan view of the absorber 50, the water absorption layer A and the water absorption layer B may be disposed to be substantially line-symmetrical with respect to the center line of the water absorption layer 1 in at least one direction. Specifically, the water absorption layer A and the water absorption layer B may be disposed to be substantially line-symmetrical with respect to the center line of the water absorption layer 1 in the longitudinal direction and/or the center line of the water absorption layer 1 in the transverse direction. Furthermore, in the water absorption layers 2 to 6(a) and 6(b) shown in Figs. 2 to 6 which will be described later, the water absorption layer A and the water absorption layer B are disposed to be substantially line-symmetrical with respect to the center line in the longitudinal direction and the center line of the water absorption layers 2 to 6(a) and 6(b) in the transverse direction.

The thickness of the water absorption layer 1 is not particularly limited, but may be, for example, 0.1 mm to 20 mm, 0.1 mm to 15 mm, 0.1 mm to 10 mm, 0.1 mm to 5 mm, 0.1 mm to 4 mm, 0.1 mm to 3 mm, 0.3 mm to 20 mm, 0.3 mm to 15 mm, 0.3 mm to 10 mm, 0.3 mm to 5 mm, 0.3 mm to 4 mm, or 0.3 mm to 3 mm. The mass per unit area of the water absorption layer 1 may be 100 g/m² to 1,000 g/m², 100 g/m² to 800 g/m², or 100 g/m² to 600 g/m². The thicknesses and/or the masses per unit area of the water absorption layer A and the water absorption layer B may be the same as or different from each other.

Fig. 2 is a top view of the water absorption layer according to an embodiment. The water absorption layer 2 shown in Fig. 2 has a strip shaped water absorption layer B that extends in the longitudinal direction of the water absorption layer 2 while including the center of the water absorption layer 2 (a portion where the center line in the longitudinal direction and the center line in the transverse direction of the water absorption layer 2 intersect), and a water absorption layer A that is disposed on both sides of the water absorption layer B in the transverse direction and extends in the longitudinal direction of the water absorption layer 2. As shown in Fig. 2, a water absorption layer other than the water absorption layer B may not be disposed on one side or both sides of the water absorption layer B in the longitudinal direction.

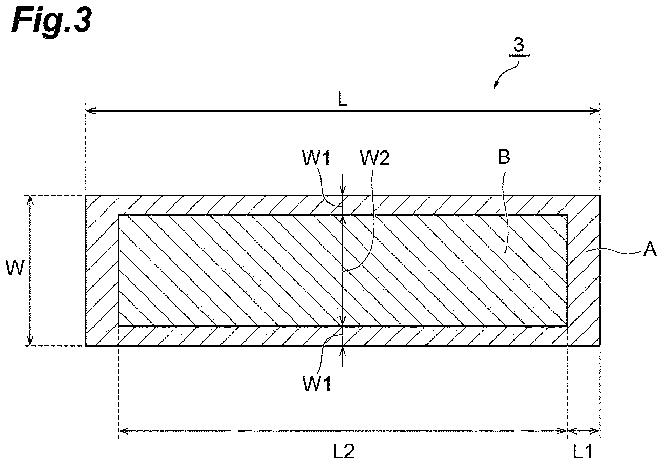

Fig. 3 is a top view of a water absorption layer according to another embodiment. The water absorption layer 3 shown in Fig. 3 has a strip shaped water absorption layer B that extends in the water absorption layer 3 in the longitudinal direction at the center of the water absorption layer 3, and a water absorption layer A that is disposed on both sides of the water absorption layer B in the transverse direction and on both sides of the water absorption layer B in the longitudinal direction and surrounds the water absorption layer B. As shown in Fig. 3, a water absorption layer other than the water absorption layer B may be disposed on one side or both sides of the water absorption layer B in the longitudinal direction.

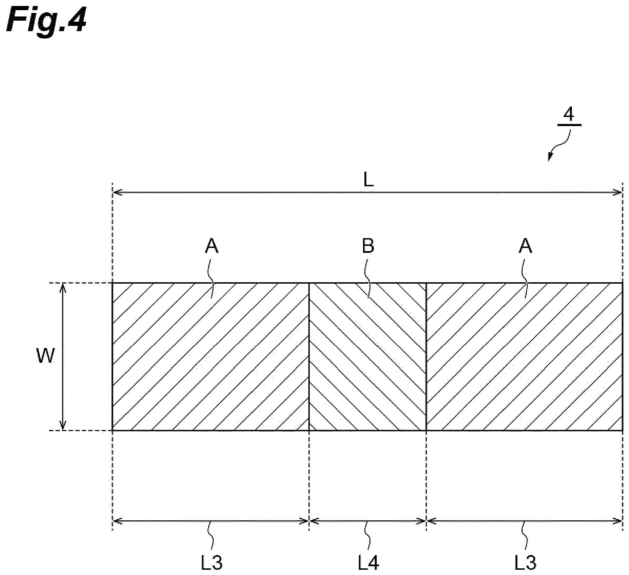

Fig. 4 is a top view of a water absorption layer according to another embodiment. The water absorption layer 4 shown in Fig. 4 has a strip shaped water absorption layer B that is disposed at the center of the water absorption layer 4 and extends in the transverse direction of the water absorption layer 4, and a water absorption layer A that is disposed on both sides of the water absorption layer B in the transverse direction and extends in the transverse direction of the water absorption layer 4. As shown in Fig. 4, a water absorption layer other than the water absorption layer B may not be disposed on one side or both sides of the water absorption layer B in the longitudinal direction.

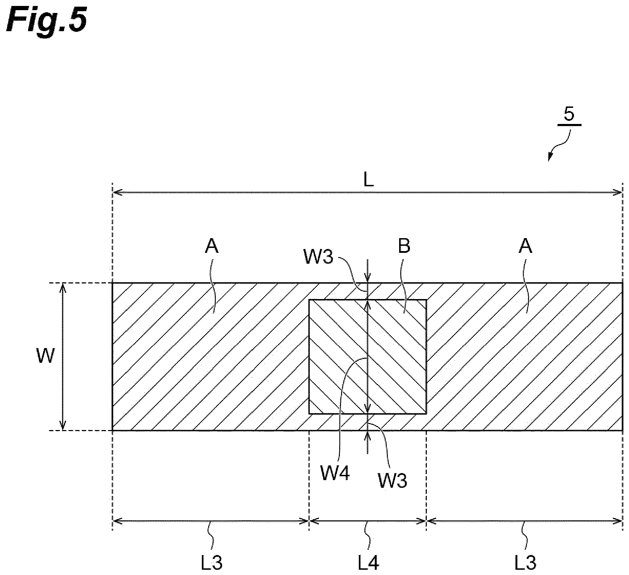

Fig. 5 is a top view of a water absorption layer according to another embodiment. The water absorption layer 5 shown in Fig. 5 has a strip shaped water absorption layer B that extends in the water absorption layer 5 in the transverse direction at the center of the water absorption layer 5, and a water absorption layer A that is disposed on both sides of the water absorption layer B in the longitudinal direction and on both sides of the water absorption layer B in the longitudinal direction and surrounds the water absorption layer B. As shown in Fig. 5, a water absorption layer other than the water absorption layer B may be disposed on one side or both sides of the water absorption layer B in the longitudinal direction.

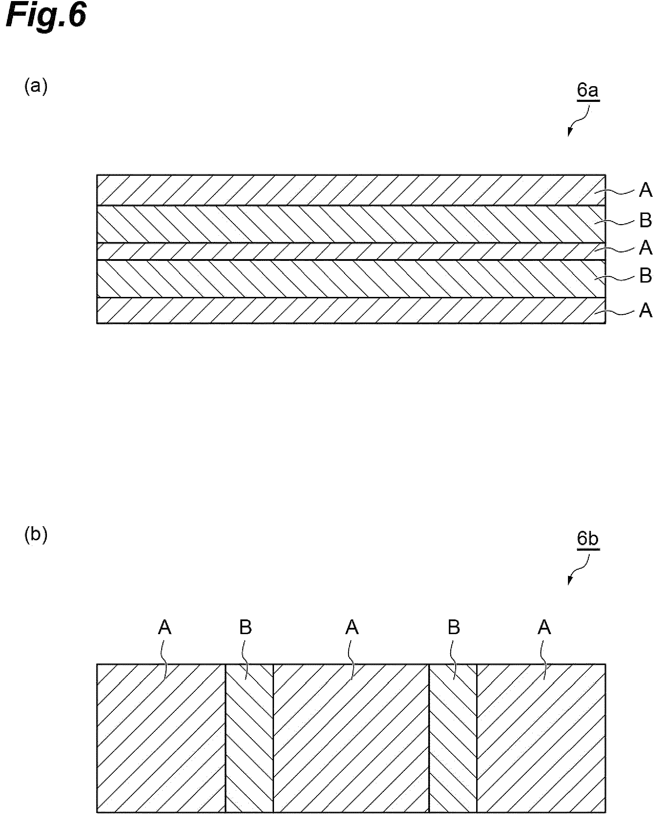

Figs. 6(a) and 6(b) are top views of the water absorption layers according to other embodiments. Both the water absorption layers 6a and 6b shown in Figs. 6(a) and 6(b) have a plurality of water absorption layers A and a plurality of water absorption layers B. The water absorption layer 6a has a plurality of strip shaped water absorption layers B that extend in the longitudinal direction of the water absorption layer 6a, and a plurality of water absorption layers A that are disposed on both sides of the water absorption layer B in the transverse direction and extend in the longitudinal direction of the water absorption layer 6a. The water absorption layer 6b has a plurality of strip shaped water absorption layers B that extend in the transverse direction of the water absorption layer 6b, and a plurality of water absorption layers A that are disposed on both sides of the water absorption layer B in the transverse direction and extend in the transverse direction of the water absorption layer 6a.

The longitudinal direction of the water absorption layers 2 and 3 is a direction extending in a front-rear direction of the groin of the user in a state of being worn by the user, and the transverse direction of the water absorption layers 2 and 3 is a direction orthogonal to the longitudinal direction of the water absorption layers 2 and 3. In a case where the water absorption layers 2 and 3 have strip shapes, the water absorption layer B may be disposed so that the longitudinal direction of the water absorption layer B is along the longitudinal direction of the water absorption layers 2 and 3, or may be disposed so that the longitudinal direction of the water absorption layer B is along the transverse direction of the water absorption layers 2 and 3. The longitudinal direction of the absorber is a direction extending in a front-rear direction of the groin of the user in a state of being worn by the user, and the transverse direction of the absorber is a direction orthogonal to the longitudinal direction of the absorber. In a case where the absorber has a strip shape, the water absorption layer B may be disposed so that the longitudinal direction of the water absorption layer B is along the longitudinal direction of the absorber, or may be disposed so that the longitudinal direction of the water absorption layer B is along the transverse direction of the absorber.

The length W of the water absorption layers 2 to 5 in the transverse direction (direction orthogonal to the longitudinal direction) may be, for example, 5 cm to 18 cm, 5 cm to 16 cm, 5 cm to 14 cm, 5 cm to 12 cm, 5 cm to 10 cm, 5 cm to 9 cm, 6 cm to 18 cm, 6 cm to 16 cm, 6 cm to 14 cm, 6 cm to 12 cm, 6 cm to 10 cm, 6 cm to 9 cm, 7 cm to 18 cm, 7 cm to 16 cm, 7 cm to 14 cm, 7 cm to 12 cm, 7 cm to 10 cm, or 7 cm to 9 cm.

The length L of the water absorption layers 2 to 5 in the longitudinal direction may be, for example, 20 cm to 60 cm, 20 cm to 50 cm, 20 cm to 40 cm, 20 cm to 38 cm, 20 cm to 36 cm, 20 cm to 34 cm, 20 cm to 32 cm, 20 cm to 30 cm, 22 cm to 60 cm, 22 cm to 50 cm, 22 cm to 40 cm, 22 cm to 38 cm, 22 cm to 36 cm, 22 cm to 34 cm, 22 cm to 32 cm, 22 cm to 30 cm, 24 cm to 60 cm, 24 cm to 50 cm, 24 cm to 40 cm, 24 cm to 38 cm, 24 cm to 36 cm, 24 cm to 34 cm, 24 cm to 32 cm, 24 cm to 30 cm, 26 cm to 60 cm, 26 cm to 50 cm, 26 cm to 40 cm, 26 cm to 38 cm, 26 cm to 36 cm, 26 cm to 34 cm, 26 cm to 32 cm, or 26 cm to 30 cm. The length of the water absorption layer B in the longitudinal direction may be the same as the length of the water absorption layer in the longitudinal direction.

On the main surfaces of the water absorption layers 2 to 5, the area of the water absorption layer A (the total area of the plurality of water absorption layers A in a case where a plurality of water absorption layers A are present) may be 30% to 99%, 30% to 97%, 30% to 95%, 30% to 90%, 50% to 99%, 50% to 97%, 50% to 95%, 50% to 90%, 60% to 99%, 60% to 97%, 60% to 95%, 60% to 90%, 70% to 99%, 70% to 97%, 70% to 95%, 70% to 90%, 75% to 99%, 75% to 97%, 75% to 95%, 75% to 90%, 80% to 99%, 80% to 97%, 80% to 95%, 80% to 90%, more than 80% and 99% or less, more than 80% and 97% or less, more than 80% and 95% or less, more than 80% and 90% or less, 85% to 99%, 85% to 97%, 85% to 95%, 85% to 90%, 90% to 99%, 90% to 97%, or 90% to 95% with respect to the total area of the water absorption layers 2 to 5.

On the main surfaces of the water absorption layers 2 to 5, the area of the water absorption layer B (the total area of the plurality of water absorption layers B in a case where a plurality of water absorption layers B are present) may be 30% or less or less than 20%, and more specifically, may be 1% to 50%, 1% to 40%, 1% to 30%, 1% or more and less than 20%, 1% to 15%, 1% to 10%, 3% to 50%, 3% to 40%, 3% to 30%, 3% or more and less than 20%, 3% to 15%, 3% to 10%, 5% to 50%, 5% to 40%, 5% to 30%, 5% or more and less than 20%, 5% to 15%, 5% to 10%, 10% to 50%, 10% to 40%, 10% to 30%, 10% or more and less than 20%, or 10% to 15% with respect to the total area of the water absorption layers 2 to 5. In a case where the area of the water absorption layer B is within the above-mentioned range, the liquid can be efficiently diffused to the entire absorber.

The length W1 of the water absorption layer A (the length between the long side of the water absorption layer A and the long side of the water absorption layer B facing the water absorption layer A) in the water absorption layers 2 and 3 in the transverse direction may be, for example, 1 cm to 8 cm, 1 cm to 7 cm, 1 cm to 6 cm, 1 cm to 5.5 cm, 2 cm to 8 cm, 2 cm to 7 cm, 2 cm to 6 cm, 2 cm to 5.5 cm, 3 cm to 8 cm, 3 cm to 7 cm, 3 cm to 6 cm, 3 cm to 5.5 cm, 4 cm to 8 cm, 4 cm to 7 cm, 4 cm to 6 cm, 4 cm to 5.5 cm, 5 cm to 8 cm, 5 cm to 7 cm, 5 cm to 6 cm, or 5 cm to 5.5 cm. The ratio (W1/W) of the length W1 of the water absorption layer A to the length W of the water absorption layers 2 and 3 in the transverse direction may be, for example, 1/12 to 6/12 or 2/12 to 5/12. Furthermore, in a case where a plurality of water absorption layers A are present, the lengths W1 of the respective water absorption layers A may be the same as or different from each other.

The length W2 of the water absorption layer B in the water absorption layers 2 and 3 in the transverse direction may be, for example, 0.5 cm to 5 cm, 0.5 cm to 4 cm, 0.5 cm to 3.5 cm, 0.5 cm to 3 cm, 0.5 cm to 2.5 cm, 0.5 cm to 2 cm, 0.5 cm to 1.5 cm, 1 cm to 5 cm, 1 cm to 4 cm, 1 cm to 3.5 cm, 1 cm to 3 cm, 1 cm to 2.5 cm, 1 cm to 2 cm, 1 cm to 1.5 cm, 1.5 cm to 5 cm, 1.5 cm to 4 cm, 1.5 cm to 3.5 cm, 1.5 cm to 3 cm, 1.5 cm to 2.5 cm, 1.5 cm to 2 cm, 2 cm to 5 cm, 2 cm to 4 cm, 2 cm to 3.5 cm, 2 cm to 3 cm, or 2 cm to 2.5 cm. Furthermore, in a case where a plurality of water absorption layers B are present, the lengths W2 of the respective water absorption layers B may be the same as or different from each other. The ratio (W2/W) of the length W2 of the water absorption layer B to the length W of the water absorption layers 2 and 3 in the transverse direction may be, for example, 1/12 to 5/12 or 1/12 to 3/12.

The length W3 of the water absorption layer A (the length between the long side of the water absorption layer A and the short side of the water absorption layer B facing the water absorption layer A) in the water absorption layer 5 in the transverse direction may be, for example, 0.5 cm to 5 cm, 0.5 cm to 4 cm, 0.5 cm to 3.5 cm, 0.5 cm to 3 cm, 0.5 cm to 2.5 cm, 0.5 cm to 2 cm, 1 cm to 5 cm, 1 cm to 4 cm, 1 cm to 3.5 cm, 1 cm to 3 cm, 1 cm to 2.5 cm, 1 cm to 2 cm, 1.5 cm to 5 cm, 1.5 cm to 4 cm, 1.5 cm to 3.5 cm, 1.5 cm to 3 cm, 1.5 cm to 2.5 cm, 1.5 cm to 2 cm, 2 cm to 5 cm, 2 cm to 4 cm, 2 cm to 3.5 cm, 2 cm to 3 cm, or 2 cm to 2.5 cm. The ratio (W3/W) of the length W3 of the water absorption layer A to the length W of the water absorption layer 5 in the transverse direction may be, for example, 1/12 to 4/12 or 2/12 to 3/12. Furthermore, in a case where a plurality of water absorption layers A are present, the lengths W3 of the respective water absorption layers A may be the same as or different from each other.

The length W4 of the water absorption layer B in the water absorption layer 5 in the transverse direction may be, for example, 4 cm to 10 cm, 4 cm to 9.5 cm, 4 cm to 9 cm, 4 cm to 9.5 cm, 4 cm to 8.5 cm, 5 cm to 10 cm, 5 cm to 9.5 cm, 5 cm to 9 cm, 5 cm to 9.5 cm, 5 cm to 8.5 cm, 6 cm to 10 cm, 6 cm to 9.5 cm, 6 cm to 9 cm, 6 cm to 9.5 cm, 6 cm to 8.5 cm, 7 cm to 10 cm, 7 cm to 9.5 cm, 7 cm to 9 cm, 7 cm to 9.5 cm, 7 cm to 8.5 cm, 8 cm to 10 cm, 8 cm to 9.5 cm, 8 cm to 9 cm, 8 cm to 9.5 cm, or 8 cm to 8.5 cm. Furthermore, in a case where a plurality of water absorption layers B are present, the lengths W4 of the respective water absorption layers B may be the same as or different from each other. The ratio (W4/W) of the length W4 of the water absorption layer B to the length W of the water absorption layer 5 in the transverse direction may be, for example, 4/12 to 10/12 or 6/12 to 8/12.

The length W2 of the water absorption layer B and the length W1 of the water absorption layer A in the water absorption layers 2 and 3 in the transverse direction, and the length W of the water absorption layers 2 and 3 in the transverse direction satisfy a relationship represented by W = W1 + W2 + W1. The length W4 of the water absorption layer B and the length W3 of the water absorption layer A in the water absorption layer 5 in the transverse direction, and the length W of the water absorption layer 5 in the transverse direction satisfy a relationship represented by W = W3 + W4 + W3.

The length L1 of the water absorption layer A (the length between the short side of the water absorption layer A and the short side of the water absorption layer B facing the water absorption layer A) in the water absorption layer 3 in the longitudinal direction may be, for example, 2 cm to 20 cm, 2 cm to 18 cm, 2 cm to 16 cm, 2 cm to 14 cm, 2 cm to 12 cm, 4 cm to 20 cm, 4 cm to 18 cm, 4 cm to 16 cm, 4 cm to 14 cm, 4 cm to 12 cm, 6 cm to 20 cm, 6 cm to 18 cm, 6 cm to 16 cm, 6 cm to 14 cm, 6 cm to 12 cm, 8 cm to 20 cm, 8 cm to 18 cm, 8 cm to 16 cm, 8 cm to 14 cm, 8 cm to 12 cm, 10 cm to 20 cm, 10 cm to 18 cm, 10 cm to 16 cm, 10 cm to 14 cm, or 10 cm to 12 cm. Furthermore, in a case where a plurality of water absorption layers A are present, the lengths L1 of the respective water absorption layers A may be the same as or different from each other. The ratio (L1/L) of the length L1 of the water absorption layer A in the water absorption layer 3 in the longitudinal direction may be, for example, 6/40 to 12/40 or 8/40 to 10/40.

The length L2 of the water absorption layer B in the water absorption layer 3 in the longitudinal direction may be, for example, 16 cm to 28 cm, 16 cm to 26 cm, 16 cm to 25 cm, 16 cm to 23 cm, 18 cm to 28 cm, 18 cm to 26 cm, 18 cm to 25 cm, 18 cm to 23 cm, 20 cm to 28 cm, 20 cm to 26 cm, 20 cm to 25 cm, or 20 cm to 23 cm. Furthermore, in a case where a plurality of water absorption layers B are present, the lengths L2 of the respective water absorption layers B may be the same as or different from each other. The ratio (L2/L) of the length L2 of the water absorption layer B in the water absorption layer 3 in the longitudinal direction may be, for example, 18/40 to 24/40 or 20/40 to 22/40.

The length L3 of the water absorption layer A (the length between the short side of the water absorption layer A and the long side of the water absorption layer B facing the water absorption layer A) in the water absorption layers 4 and 5 in the longitudinal direction may be, for example, 10 cm to 26 cm, 10 cm to 24 cm, 10 cm to 22 cm, 10 cm to 20 cm, 12 cm to 26 cm, 12 cm to 24 cm, 12 cm to 22 cm, 12 cm to 20 cm, 14 cm to 26 cm, 14 cm to 24 cm, 14 cm to 22 cm, 14 cm to 20 cm, 16 cm to 26 cm, 16 cm to 24 cm, 16 cm to 22 cm, 16 cm to 20 cm, 18 cm to 26 cm, 18 cm to 24 cm, 18 cm to 22 cm, or 18 cm to 20 cm. Furthermore, in a case where a plurality of water absorption layers A are present, the lengths L3 of the respective water absorption layers A may be the same as or different from each other. The ratio (L3/L) of the length L3 of the water absorption layer A to the water absorption layers 4 and 5 in the longitudinal direction may be, for example, 16/40 to 24/40 or 18/40 to 22/40.

The length L4 of the water absorption layer B in the water absorption layers 4 and 5 in the longitudinal direction may be, for example, 0.5 cm to 6 cm, 0.5 cm to 5 cm, 0.5 cm to 4 cm, 0.5 cm to 3 cm, 1 cm to 6 cm, 1 cm to 5 cm, 1 cm to 4 cm, 1 cm to 3 cm, 1.5 cm to 6 cm, 1.5 cm to 5 cm, 1.5 cm to 4 cm, 1.5 cm to 3 cm, 2 cm to 6 cm, 2 cm to 5 cm, 2 cm to 4 cm, or 2 cm to 3 cm. Furthermore, in a case where a plurality of water absorption layers B are present, the lengths L4 of the respective water absorption layers B may be the same as or different from each other. The ratio (L4/L) of the length L4 of the water absorption layer B to the water absorption layers 4 and 5 in the longitudinal direction may be, for example, 1/40 to 6/40 or 1/40 to 4/40.

### (Other Components That Can Be Included in Water Absorption Layer)

The water absorption layer A may or may not include a fibrous substance 11a. Similarly, the water absorption layer B may or may not include a fibrous substance 11b. In a case where the water absorption layer A and/or the water absorption layer B includes the fibrous substance 11a and/or the fibrous substance 11b, the content of the fibrous substances in each water absorption layer may be more than 0% by mass and 10% by mass or less, more than 0% by mass and 8% by mass or less, more than 0% by mass and 5% by mass or less, more than 0% by mass and 3% by mass or less, or more than 0% by mass and 2% by mass or less with respect to the mass of each water absorption layer. The content of the fibrous substance 11a in the water absorption layer A and the content of the fibrous substance 11b in the water absorption layer B may be the same as or different from each other.

The fibrous substances 11a and 11b can be, for example, cellulosic fibers, synthetic fibers, or a combination thereof. Examples of the cellulosic fibers include pulverized wood pulp, cotton, cotton linter, rayon, and cellulose acetate. Examples of the synthetic fibers include polyamide fibers, polyester fibers, and polyolefin fibers. The fibrous substances may be hydrophilic fibers (for example, pulp).

The water absorption layer A and/or the water absorption layer B may further include additives such as inorganic particles (for example, amorphous silica), a deodorant, an antibacterial agent, and a fragrance. In a case where the water-absorbent resin particles include the inorganic particles, the water absorption layer A and/or the water absorption layer B may include inorganic particles separately from the inorganic particles in the water-absorbent resin particles. In a case where the water absorption layer A and/or the water absorption layer B includes these additives, the content of the additives in each water absorption layer may be more than 0% by mass and 2% by mass or less, more than 0% by mass and 1% by mass or less, or more than 0% by mass and 0.5% by mass or less with respect to the mass of each water absorption layer. The content of these additives in the water absorption layer A and the content of these additives in the water absorption layer B may be the same as or different from each other.

### [Core Wrap Sheet]

Core wrap sheets 20a and 20b in the absorber 50 may be, for example, non-woven fabrics. The two core wrap sheets 20a and 20b can be the same or different non-woven fabrics. The non-woven fabric may be a non-woven fabric (short-fiber non-woven fabric) formed of short fibers (that is, staples), or may be a non-woven fabric (long-fiber non-woven fabric) formed of long fibers (that is, filaments). In general, staples may have a fiber length of several hundred millimeters or shorter, but there is no limitation.

The core wrap sheets 20a and 20b may be thermal bonded non-woven fabrics, air-through non-woven fabrics, resin bonded non-woven fabrics, spunbond non-woven fabrics, melt-blown non-woven fabrics, air-laid non-woven fabrics, spunlace non-woven fabrics, point-bonded non-woven fabrics, or a laminate containing two or more of non-woven fabrics selected from these non-woven fabrics.

The non-woven fabric used as the core wrap sheets 20a and 20b can be a non-woven fabric formed of synthetic fibers, natural fibers, or a combination thereof. Examples of the synthetic fibers include fibers including synthetic resins selected from polyolefin such as polyethylene (PE) and polypropylene (PP); polyester such as polyethylene terephthalate (PET), polytrimethylene terephthalate (PTT), and polyethylene naphthalate (PEN); polyamide such as nylon; and rayon. Examples of the natural fibers include fibers including cotton, silk, hemp, or pulp (cellulose). Fibers forming the non-woven fabric may be polyolefin fibers, polyester fibers, or a combination thereof. The core wrap sheets 20a and 20b may be tissue paper.

The core wrap sheets 20a and 20b may have a main surface wider than the main surface of the water absorption layer 1. That is, the outer edge portions of the core wrap sheets 20a and 20b may extend around the water absorption layer 1. The core wrap sheets 20a and 20b may be bonded to each other at the outer edge portion extending around the water absorption layer 1.

### [Adhesive]

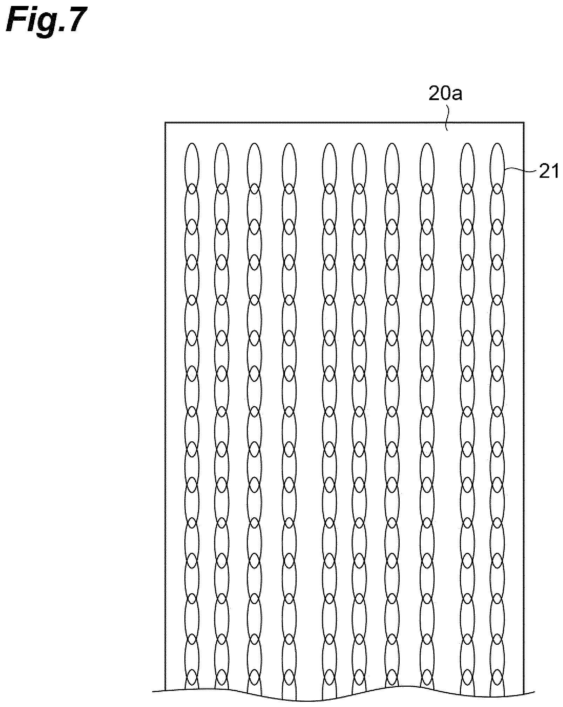

The absorber 50 may further have an adhesive layer 21 sandwiched between the core wrap sheets 20a and 20b and the water absorption layer 1. Fig. 7 is a plan view showing an example of a pattern of an adhesive, formed on a core wrap sheet. An adhesive layer 21 shown in Fig. 7 forms a pattern composed of a plurality of linear portions disposed with intervals therebetween on the core wrap sheet 20a. It should be noted that the pattern of the adhesive layer 21 is not limited thereto. The adhesive may be sandwiched only between any one of the core wrap sheets 20a and 20b and the water absorption layer 1. The adhesive layer 21 may be formed of, for example, a hot melt adhesive.

### <Estimated Mechanism of Effect>

In the absorber of the present invention, in a case where a liquid to be absorbed comes into contact with the water absorption layer, the liquid penetrates into the water absorption layer. The liquid that has penetrated into the water absorption layer swells the water-absorbent resin particles included in the water absorption layer. However, in the present invention, since the 1-minute value of the lock-up height of the water absorption layer A is sufficiently larger than the 1-minute value of the lock-up height of the water absorption layer B, the water absorption layer A swells more quickly and to a greater extent than the water absorption layer B. As a result, the water absorption layer A disposed on both sides of the water absorption layer B in the transverse direction is more likely to be bulged than the water absorption layer B, and a groove portion formed of the water absorption layer B is formed between the two water absorption layers A. It is considered that the liquid reaching the water absorption layer B from the main surface of the absorber flows along the groove portion without being retained in one place, thereby suppressing the occurrence of a local gel blocking phenomenon in the water absorption layer B and the water absorption layers A located on both sides thereof. In addition, the water absorption layer having a high swelling power tends to cause a gel blocking phenomenon. However, in the present invention, the swelling power of the water absorption layer A is set to be less than 5 N in terms of the 10-second value, the occurrence of the gel blocking phenomenon is suppressed in the water absorption layer A, and it is thus considered that the liquid flowing along the groove portion (water absorption layer B) efficiently permeates the entire water absorption layer A. As described above, in the present invention, the entire absorber is efficiently used to easily absorb the liquid in the water absorption layer 1, and it is thus considered that the absorber of the present invention and the absorbent article formed of the absorber have a high permeation rate and a large absorption capacity.

### <Absorbent Article>

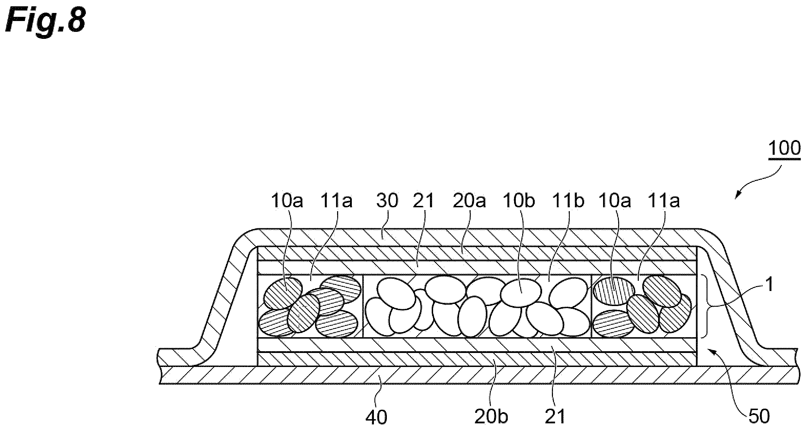

Fig. 8 is a cross-sectional view showing an example of an absorbent article. An absorbent article 100 shown in Fig. 8 includes an absorber 50, a liquid permeable sheet 30, and a liquid impermeable sheet 40. In the absorbent article 100, the absorber 50 is sandwiched between the liquid permeable sheet 30 and the liquid impermeable sheet 40. Examples of the absorbent article 100 having such a configuration include a paper diaper, an incontinence pad, a sanitary napkin, a pet sheet, and a food drip sheet.

The liquid permeable sheet 30 is disposed at the position of the outermost layer on the side where the liquid to be absorbed enters. The liquid permeable sheet 30 is disposed on the outer side of the core wrap sheets 20a and 20b in a state of being in contact with the core wrap sheets 20a and 20b. The liquid impermeable sheet 40 is disposed at the position on the outermost layer on the opposite side to the liquid permeable sheet 30 in the absorbent article 100. The liquid impermeable sheet 40 is disposed on an outer side of the core wrap sheet 20a in a state of being in contact with the core wrap sheet 20a. The liquid permeable sheet 30 and the liquid impermeable sheet 40 have a main surface wider than the main surface of the absorber 50, and outer edge portions of the liquid permeable sheet 30 and the liquid impermeable sheet 40 extend around the water absorption layer 1 and the core wrap sheets 20a and 20b. However, the magnitude relationship among the water absorption layer 1, the core wrap sheets 20a and 20b, the liquid permeable sheet 30, and the liquid impermeable sheet 40 is not particularly limited, and is appropriately adjusted according to a use of the absorbent article, or the like.

The liquid permeable sheet 30 may be a non-woven fabric. The non-woven fabric used as the liquid permeable sheet 30 may have appropriate hydrophilicity from the viewpoint of the liquid absorption performance of the absorbent article. From this viewpoint, the liquid permeable sheet 30 may be a non-woven fabric having a hydrophilicity of 5 to 200 measured according to a measurement method of Pulp and Paper Test Method No. 68 (2000) by the Japan Technical Association of Pulp and Paper Industry. The hydrophilicity of the non-woven fabric may be 10 to 150. For details of the paper pulp test method No. 68, for example, reference can be made to WO2011/086843A.

The non-woven fabric having hydrophilicity may be formed of fibers, such as rayon fibers, showing appropriate hydrophilicity, or may be formed of fibers obtained by hydrophilizing hydrophobic chemical fibers such as polyolefin fibers and polyester fibers. Examples of a method of obtaining a non-woven fabric containing hydrophobic chemical fibers that have been hydrophilized include a method of obtaining a non-woven fabric by a spunbond technique using one obtained by mixing a hydrophilizing agent with hydrophobic chemical fibers, a method of using a hydrophilizing agent in a case of preparing a spunbond non-woven fabric with hydrophobic chemical fibers, and a method of impregnating a spunbond non-woven fabric obtained using hydrophobic chemical fibers with a hydrophilizing agent. As the hydrophilizing agent, for example, anionic surfactants such as aliphatic sulfonic acid salts and higher alcohol sulfuric acid ester salts; cationic surfactants such as quaternary ammonium salts; nonionic surfactants such as polyethylene glycol fatty acid esters, polyglycerin fatty acid esters, and sorbitan fatty acid esters; silicone surfactants such as polyoxyalkylene-modified silicone; stain release agents formed of polyester-based, polyamide-based, acrylic-based, or urethane-based resin; or the like are used.

From the viewpoint of imparting good liquid permeability, flexibility, strength, and cushioning properties to the absorbent article, and the viewpoint of accelerating a permeation rate of the absorbent article, the basis weight (mass per unit area) of the non-woven fabric used as the liquid permeable sheet 30 may be 5 to 200 g/m², 8 to 150 g/m², or 10 to 100 g/m². The thickness of the liquid permeable sheet 30 may be 20 to 1,400 µm, 50 to 1,200 µm, or 80 to 1,000 µm.

The liquid impermeable sheet 40 prevents a liquid absorbed by the water absorption layer 1 from leaking to the outside from the liquid impermeable sheet 40 side. The liquid impermeable sheet 40 may be a resin sheet or a non-woven fabric. The resin sheet may be a sheet made of a synthetic resin such as polyethylene, polypropylene, and polyvinyl chloride. The non-woven fabric may be a spunbond/melt-blown/spunbond (SMS) non-woven fabric in which a water-resistant melt-blown non-woven fabric is sandwiched between high-strength spunbond non-woven fabrics. The liquid impermeable sheet 40 may be a composite sheet of a resin sheet and a non-woven fabric (for example, spunbond non-woven fabric, spunlace non-woven fabric). The liquid impermeable sheet 40 may have breathability from the viewpoint that dampness generated at the time of wearing is reduced, and thereby discomfort to a wearer can be reduced. As the liquid impermeable sheet 40 having breathability, it is possible to use a sheet of a low-density polyethylene (LDPE) resin, for example.

From the viewpoint of ensuring flexibility not to impair a sense of wearing the absorbent article, the basis weight (mass per unit area) of the liquid impermeable sheet 40 may be 10 to 50 g/m².

The absorbent article 100 can be produced, for example, by a method including disposing the absorber 50 between the liquid permeable sheet 30 and the liquid impermeable sheet 40. The laminate obtained by laminating the liquid impermeable sheet 40, the absorber 50, and the liquid permeable sheet 30 in this order is pressurized as necessary. Alternatively, the absorbent article 100 can be obtained by a method in which the liquid permeable sheet 30, the core wrap sheets 20a and 20b, the water-absorbent resin particles 10a or a mixture including the water-absorbent resin particles 10a and the fibrous substance, the core wrap sheet 20a, and the liquid impermeable sheet 40 are disposed in this order, and the formed structure is pressurized while heating as necessary.

### Examples

Hereinafter, the present disclosure will be more specifically described with reference to Examples. It should be noted that the present invention is not limited to these Examples.

### <Production of Water-Absorbent Resin Particles>

### (Production Example 1)

### <First Stage Polymerization Reaction>

A round-bottomed cylindrical separable flask having an inner diameter of 11 cm and a volume of 2L and equipped with a reflux cooling device, a dropping funnel, a nitrogen gas introduction tube, and a stirrer (a stirrer blade having two stages of four inclined paddle blades with a blade diameter of 5 cm) was prepared. 293 g of n-heptane (hydrocarbon dispersion medium) and 0.736 g of a maleic anhydride-modified ethylene/propylene copolymer (Hi-Wax 1105A, Mitsui Chemicals, Inc., polymeric dispersant) were added to this separable flask and mixed to obtain a mixture. The dispersant was dissolved by heating the mixture to 80°C while stirring the mixture at a rotation speed of 300 rpm. Thereafter, the mixture was cooled to 55°C.

Subsequently, 92.0 g of an aqueous solution of 80.5% by mass acrylic acid (acrylic acid: 1.03 mol) as a water-soluble ethylenically unsaturated monomer was put to a triangular flask having a volume of 300 mL. Next, while the mixture was cooled from the outside, 75% by mole of acrylic acid was neutralized by adding 147.7 g of a 20.9% by mass aqueous sodium hydroxide solution dropwise to the mixture. Thereafter, 0.092 g of hydroxyethyl cellulose (thickener, Sumitomo Seika Chemicals Co., Ltd., HEC AW-15F), 0.0736 g (0.272 mmol) of potassium persulfate (water-soluble radical polymerization initiator), and 0.010 g (0.057 mmol) of ethylene glycol diglycidyl ether (internal crosslinking agent) were added thereto and dissolved to prepare a first stage aqueous monomer solution.

The above-mentioned first stage aqueous monomer solution was added to the above-mentioned n-heptane solution including the dispersant in the separable flask, and then the mixture was stirred for 10 minutes. Separately, 0.736 g of a sucrose stearic acid ester (surfactant, manufactured by Mitsubishi-Chemical Foods Corporation, Ryoto Sugar Ester S-370, HLB: 3) was heat-dissolved in 6.62 g of n-heptane to obtain a surfactant solution. 7.356 g of the obtained surfactant solution was added to the separable flask to obtain a reaction solution. Next, the inside of the separable flask system was thoroughly substituted with nitrogen while the reaction solution was stirred at a rotation speed of 550 rpm. Thereafter, the separable flask was immersed in a water bath at 70°C to heat the reaction solution and first stage polymerization was performed for 60 minutes to obtain a first stage reaction mixture.

### <Second Stage Polymerization Reaction>

Subsequently, 128.8 g (acrylic acid: 1.44 mol) of an 80.5% by mass aqueous acrylic acid solution was put into another triangular flask having a volume of 500 mL. Next, while the mixture was cooled from the outside, 75% by mole of acrylic acid was neutralized by adding 159.0 g of a 27% by mass aqueous sodium hydroxide solution dropwise to the mixture. Thereafter, 0.1030 g (0.3810 mmol) of potassium persulfate (water-soluble radical polymerization initiator) and 0.0116 g (0.0666 mmol) of ethylene glycol diglycidyl ether (internal crosslinking agent) were added thereto and dissolved to prepare a second stage aqueous monomer solution.

After the first stage reaction mixture was cooled to 25°C while being stirred at a rotation speed of 1000 rpm, the total amount of the second stage monomer aqueous solution was added to the first stage reaction mixture to obtain a reaction solution. Next, the inside of the system was sufficiently substituted with nitrogen while the reaction solution was stirred. Thereafter, the separable flask was immersed in a water bath at 70°C to heat the reaction solution and second stage polymerization was performed for 5 minutes to obtain a second stage reaction mixture (polymer particles before surface crosslinking).

After the second stage polymerization, the second stage reaction mixture was heated in an oil bath at 125°C and 255 g of water was extracted to the outside of the system by azeotropic distillation of n-heptane and water while the n-heptane was refluxed. Subsequently, 0.0884 g (0.5075 mmol) of ethylene glycol diglycidyl ether was added as a surface crosslinking agent and then the result was held at 83°C for 2 hours to obtain a dispersion liquid of polymer particles after surface crosslinking.

Thereafter, the dispersion liquid of the polymer particles after the above-mentioned surface crosslinking was heated in an oil bath at 125°C, and n-heptane was evaporated and dried, thereby obtaining polymer particles (dried product). The polymer particles were allowed to pass through a sieve having an opening of 850 µm, thereby obtaining 223.4 g of water-absorbent resin particles (1) in a form in which spherical particles were aggregated.

### (Production Example 2)

0.2% by mass of amorphous silica (TOKUSIL NP-S, Oriental Silicas Corporation, hydrophilic) with respect to the mass of water-absorbent resin particles (1) was mixed with the water-absorbent resin particles (1) to obtain water-absorbent resin particles (2) (water-absorbent resin particles A).

### (Production Example 3)

225.1 g of water-absorbent resin particles (3) (water-absorbent resin particles B) was obtained in the same manner as in Production Example 1, except that the amount of water extracted to the outside of the system by azeotropic distillation was changed to 237 g, and 0.1% by mass of amorphous silica (Oriental Silicas Corporation, TOKUSIL NP-S, hydrophilic) with respect to the mass of the water-absorbent resin particles (1) was mixed with the water-absorbent resin particles (1).

### (Production Example 4)

224.2 g of water-absorbent resin particles (4) (water-absorbent resin particles C) was obtained in the same manner as in Production Example 1, except that the amount of water extracted to the outside of the system by azeotropic distillation was changed to 246 g, and 0.1% by mass of amorphous silica (Oriental Silicas Corporation, TOKUSIL NP-S, hydrophilic) with respect to the mass of the water-absorbent resin particles (1) was mixed with the water-absorbent resin particles (1).

### (Production Example 5)

227.2 g of water-absorbent resin particles (5) (water-absorbent resin particles D) were obtained in the same manner as in Production Example 1, except that 0.092 g (0.339 mmol) of 2,2'-azobis(2-amidinopropane) dihydrochloride as the azo-based compound was added to the first stage aqueous monomer solution, the addition amount of potassium persulfate was changed to 0.028 g (0.102 mmol), the addition amount of ethylene glycol diglycidyl ether was changed to 0.0101 g (0.0580 mmol), 0.129 g (0.475 mmol) of 2,2'-azobis(2-amidinopropane) dihydrochloride as the azo-based compound was added to the second stage aqueous monomer solution, the addition amount of potassium persulfate was changed to 0.039 g (0.143 mmol), the amount of water to be extracted to the outside of the system by azeotropic distillation was changed to 257.5 g, 0.1104 g (0.6338 mmol) of ethylene glycol diglycidyl ether was added as a surface crosslinking agent, and the mixing amount of the amorphous silica with respect to the water-absorbent resin particles was changed to 0.2% by mass.

### (Production Example 6)

From a commercially available product of baby diapers (manufactured by Hengan International Group Co., Ltd., product name: Q MO Natural Cotton, tape type, L size), only the upper layer side was taken out from an absorber sheet composed of the water-absorbent resin particles and the non-woven fabric, the air-laid non-woven fabric of the upper layer and the air-through non-woven fabric of the intermediate layer were removed to obtain 50.42 g of the water-absorbent resin particles (6) (water-absorbent resin particles E) having an irregular shape, which were presumed to be manufactured by an aqueous solution polymerization method.

### (Production Example 7)

An absorber mixture composed of water-absorbent resin particles and pulp was taken out from a commercially available product of baby diapers (product name: Merries Pants First Premium, manufactured by Kao Corporation, pant type, L size), and the pulp was removed to obtain 53.46 g of water-absorbent resin particles (7) (water-absorbent resin particles F) having an irregular shape, which were presumed to be manufactured by an aqueous solution polymerization method.

### (Production Example 8)

Production Example 1 was repeated, and the collected water-absorbent resin particles (1) were classified with a sieve having an opening of 250 µm to obtain 500.0 g of water-absorbent resin particles (8) having a particle diameter of 250 to 850 µm.

### (Production Example 9)

### [Step of Producing Emulsion Material]

Water and ice were added to a plastic bat having a length of 27 cm, a width of 38 cm, and a depth of 7 cm to manufacture an ice bath at 3°C. A glass beaker having an internal volume of 1 L was placed in this ice bath and 897.41 g of ion-exchanged water was added thereto. The ice bath was placed on top of a magnetic stirrer, a stirrer tip was added to the inside of the beaker, and the mixture was stirred.

10.55 g of sodium hydroxide (granules) (Nacalai Tesque Inc.) was added little by little to a beaker to manufacture an aqueous sodium hydroxide solution.

A round-bottomed cylindrical separable flask having an inner diameter of 11 cm and an internal volume of 2 L and equipped with a reflux cooling device, a thermometer, and a stirrer (stirrer blades having four inclined paddle blades having a blade diameter of 5 cm) was prepared. 100 g of an ethylene-acrylic acid copolymer (SK Global Chemical: Primacor 5980i) was added to this flask. Thereafter, the total amount of the above-mentioned aqueous sodium hydroxide solution was added thereto. Thereafter, the beaker used to manufacture the aqueous sodium hydroxide solution was washed with 50.0 g of ion-exchanged water and the washing water was added into the separable flask to obtain a reaction solution.

While stirring with a stirrer at a rotation speed of 500 rpm, the reaction solution was immersed in an oil bath at 103°C and the internal temperature was raised to 95°C. Thereafter, the temperature of the oil bath was appropriately adjusted such that the internal temperature corresponded to 95°C to 97°C, which was maintained for 4 hours.

The separable flask was pulled up from the oil bath and left to be cooled to room temperature until the internal temperature reached 35°C. After confirming that the internal temperature was 35°C or lower, the reaction solution was filtered through a nylon mesh having an opening of 108 µm to obtain a 10% water-dispersed emulsion material of an ethylene-sodium acrylate copolymer having a degree of neutralization of 95% as the filtrate. Furthermore, the ratio of the monomeric units derived from ethylene in the ethylene-sodium acrylate copolymer was 91.1% by mole.

### [Step of Manufacturing Coating Agent]

500.0 g of a 10% water-dispersed emulsion material of an ethylene-sodium acrylate copolymer having a degree of neutralization of 95% and 5.0 g of polyethylene glycol (PEG6000, Tokyo Chemical Industry Co., Ltd.) having a number-average molecular weight of 6,000 were mixed in a polybeaker having an internal volume of 1 L to prepare a coating agent (1).

### [Step of Producing Water-Absorbent Resin Particles Having Coating Layer]

500.0 g of water-absorbent resin particles (8) were injected into the container of a fluidized bed granulator, and hot air at 50°C was blown from the bottom of the container. Subsequently, while drying, 505 g of the coating agent (1) was sprayed onto the water-absorbent resin particles, which were whirled up by blowing. After spraying the coating agent, the coating agent was dried at 50°C for 30 minutes to obtain covered resin particles.

50.0 g of the obtained covered resin particles were spread in a metal bat measuring 26 cm in length and 20 cm in width, and covered with aluminum foil. The aluminum foil was perforated and the covered resin particles were heated for 60 minutes by a hot air dryer (ADVANTEC, FV-320) set at 100°C to obtain 50.0 g of water-absorbent resin particles (9) (water-absorbent resin particles G) having a coating layer.

### (Production Example 10)

50.0 g of water-absorbent resin particles (10) (water-absorbent resin particles H) having a coating layer was obtained in the same manner as in Production Example 9, except that in the step of manufacturing the coating agent, 1,000.0 g of a 10% water-dispersed emulsion material of an ethylene-sodium acrylate copolymer having a degree of neutralization of 95% and 25.0 g of polyethylene glycol (Tokyo Chemical Industry Co., Ltd., PEG6000) were mixed to prepare a coating agent (2), the coating agent (2) was used instead of the coating agent (1), the spray amount of the coating agent was changed to 1,025 g, and the set temperature of the hot air dryer was changed to 90°C.

### (Production Example 11)

50.0 g of water-absorbent resin particles (11) (water-absorbent resin particles I) having a coating layer was obtained in the same manner as in Production Example 9, except that in the step of manufacturing the coating agent, 1,500.0 g of a 10% water-dispersed emulsion material of an ethylene-sodium acrylate copolymer having a degree of neutralization of 95% as an emulsion material and 25.0 g of polyethylene glycol (Tokyo Chemical Industry Co., Ltd., PEG6000) were mixed to prepare a coating agent (3), the coating agent (3) was used instead of the coating agent (1), and the spray amount of the coating agent was changed to 1,525 g.

### (Production Example 12)

### <First Stage Polymerization Reaction>

A round-bottomed cylindrical separable flask having an inner diameter of 11 cm and a volume of 2 L and equipped with a reflux cooling device, a dropping funnel, a nitrogen gas introduction tube, and a stirrer (a stirrer blade having two stages of four inclined paddle blades with a blade diameter of 5 cm) was prepared. 293 g of n-heptane (hydrocarbon dispersion medium) and 0.736 g of a maleic anhydride-modified ethylene-propylene copolymer (polymeric dispersant, Mitsui Chemicals, Inc., Hi-Wax 1105A) were added to this separable flask to obtain a mixture. The dispersant was dissolved by heating the mixture to 80°C while stirring the mixture at a rotation speed of 300 rpm. Thereafter, the mixture was cooled to 50°C.

Subsequently, 92.0 g of a 80.5% by mass aqueous acrylic acid solution (acrylic acid: 1.03 mol) was put into a triangular flask having a volume of 300 mL. Next, while the mixture was cooled from the outside, 75% by mole of acrylic acid was neutralized by adding 147.7 g of a 20.9% by mass aqueous sodium hydroxide solution dropwise to the mixture. Subsequently, 0.092 g of hydroxyethyl cellulose (HEC AW-15F, SUMITOMO SEIKA CHEMICALS CO., LTD., thickener), 0.0736 g (0.272 mmol) of potassium persulfate (water-soluble radical polymerization initiator), and 0.010 g (0.057 mmol) of ethylene glycol diglycidyl ether (internal crosslinking agent) were added thereto and dissolved to prepare a first stage aqueous monomer solution.

The above-mentioned first stage aqueous monomer solution was added to the above-mentioned n-heptane solution including the dispersant in the separable flask, and then the mixture was stirred for 10 minutes. Separately, 0.736 g of a sucrose stearic acid ester (surfactant, manufactured by Mitsubishi-Chemical Foods Corporation, Ryoto Sugar Ester S-370, HLB: 3) was heat-dissolved in 6.62 g of n-heptane to obtain a surfactant solution. 7.356 g of the obtained surfactant solution to the separable flask to obtain a reaction solution. Next, the inside of the separable flask system was thoroughly substituted with nitrogen while the reaction solution was stirred at a rotation speed of 550 rpm. Thereafter, the separable flask was immersed in a water bath at 70°C to heat the reaction solution and first stage polymerization was performed for 60 minutes to obtain a first stage reaction mixture.

### <Second Stage Polymerization Reaction>

Subsequently, 128.8 g (acrylic acid: 1.44 mol) of an 80.5% by mass aqueous acrylic acid solution was put into another triangular flask having a volume of 500 mL. Next, while the mixture was cooled from the outside, 75% by mole of acrylic acid was neutralized by adding 159.0 g of a 27% by mass aqueous sodium hydroxide solution dropwise to the mixture. Thereafter, 0.090 g (0.333 mmol) of potassium persulfate and 0.0116 g (0.0666 mmol) of ethylene glycol diglycidyl ether (internal crosslinking agent) were added thereto and then dissolved, thereby preparing a second stage aqueous monomer solution.

After the first stage reaction mixture was cooled to 25°C while being stirred at a rotation speed of 1000 rpm, the total amount of the second stage aqueous monomer solution was added to the first stage reaction mixture to obtain a reaction solution. Next, the inside of the system was sufficiently substituted with nitrogen while the reaction solution was stirred. Thereafter, the separable flask was immersed in a water bath at 70°C to heat the reaction solution and second stage polymerization was performed for 5 minutes to obtain a second stage reaction mixture (polymer particles before surface crosslinking).

After the second stage polymerization, 0.265 g of a 45% by mass aqueous pentasodium diethylenetriaminepentaacetate solution was added thereto under stirring. Thereafter, the temperature of the second stage reaction mixture was raised in an oil bath at 125°C, and 272 g of water was extracted to the outside of the system while refluxing n-heptane by azeotropic distillation of n-heptane and water. Subsequently, 0.128 g (0.735 mmol) of ethylene glycol diglycidyl ether was added as a surface crosslinking agent and then the result was held at 83°C for 2 hours to obtain a dispersion liquid of polymer particles after surface crosslinking.

Thereafter, the dispersion liquid of the polymer particles after the above-mentioned surface crosslinking was heated in an oil bath at 125°C, and n-heptane was evaporated and dried, thereby obtaining polymer particles (dried product). The polymer particles were classified with sieves having an opening size of 850 µm and an opening size of 250 µm to obtain 220.2 g of water-absorbent resin particles (12) having a particle diameter of 250 to 850 µm.

### (Production Example 13)

50.0 g of water-absorbent resin particles (13) (water-absorbent resin particles J) having a coating layer was obtained by the same manner as in Production Example 10, except that 500 g of the water-absorbent resin particles (12) of Production Example 12 was used instead of the water-absorbent resin particles (8).

### <Evaluation of Water-Absorbent Resin Particles>

### [Water Retention Amount of Physiological Saline]

After adding 500 g of physiological saline at 25°C to a 500 mL polyethylene beaker, 2.00 g of water-absorbent resin particles for evaluation was added little by little while rotating a stirring element (8 mm × 30 mm, no ring) at a rotation speed of 600 rpm using a stirrer, and after the total amount of the water-absorbent resin particles was added thereto, the mixture was stirred for 30 minutes. Next, after the mixture was transferred into a cotton bag (Cotton broadcloth No. 60, 100 mm in width × 200 mm in length), the upper part of the cotton bag was closed with a rubber band. Subsequently, the mixture was dehydrated for 1 minute using a centrifuge (manufactured by KOKUSAN Co., Ltd., product number: H-122) which had been set to have a centrifugal force of 167 G, and the mass Wa [g] of the cotton bag including the swollen gel after dehydration was measured. The same operation was performed without addition of the water-absorbent resin particles, the empty mass Wb [g] at the time in a case where the cotton bag was wet was measured, and the water retention amount (room temperature) of the physiological saline of the water-absorbent resin particles was calculated from the following equation. The measurement results of the water retention amount of physiological saline water are shown in Table 1. Water retention amount of physiological saline [g/g] = (Wa - Wb)/Amount of water-absorbent resin particles

### [Water Absorption Rate (Vortex Method)]

50.0 g of physiological saline was added into a 100 mL beaker having a stirring element (8 mm × 30 mm, no ring) and maintained at 25°C in a constant-temperature tank. Subsequently, 2.00 g of water-absorbent resin particles for evaluation were injected into a vortex of the physiological saline stirred at 600 rpm to start measurement with a stopwatch at the same time. The time point when the vortex disappeared and the liquid level became horizontal was denoted as an end point, and the time (seconds) until the end point was denoted as the water absorption rate. The measurement results of the water absorption rate are shown in Table 1.

### [Lock-Up Height]

The lock-up heights of the water-absorbent resin particles A to J and the water absorption layers A and B were measured by a method shown below. 0.200 g of water-absorbent resin particles for evaluation (collected from the water absorption layers A and B in a case of measuring the lock-up height of the water absorption layers A and B) was accurately weighed, and layered on the bottom of an acrylic cylinder having an inner diameter of 2.0 cm and a depth of 8.0 cm to form a particle layer having a flat upper surface, and then the height H0 [cm] of the particle layer was measured. Then, 20 g of physiological saline at 25°C was poured from the upper part of the acrylic cylinder at once. The measurement was started from the time point when the total amount of physiological saline was put in, the height H1 [cm] of the particle layer after 1 minute was read, and the lock-up height was calculated from the following equation. Furthermore, in a case where the upper surface of the particle layer after the water absorption was not flat, the height of the highest portion was denoted as H1. The measurement results of the lock-up height are shown in Table 1. In addition, the lock-up height of each of the water absorption layer A and the water absorption layer B of the absorbent articles of Examples and Comparative Examples which will be described later, and the difference between the lock-up heights are shown in Table 4. Lock-up height [cm/g] = (H1 - H0)/(Amount of water-absorbent resin particles)

### [Swelling Power]

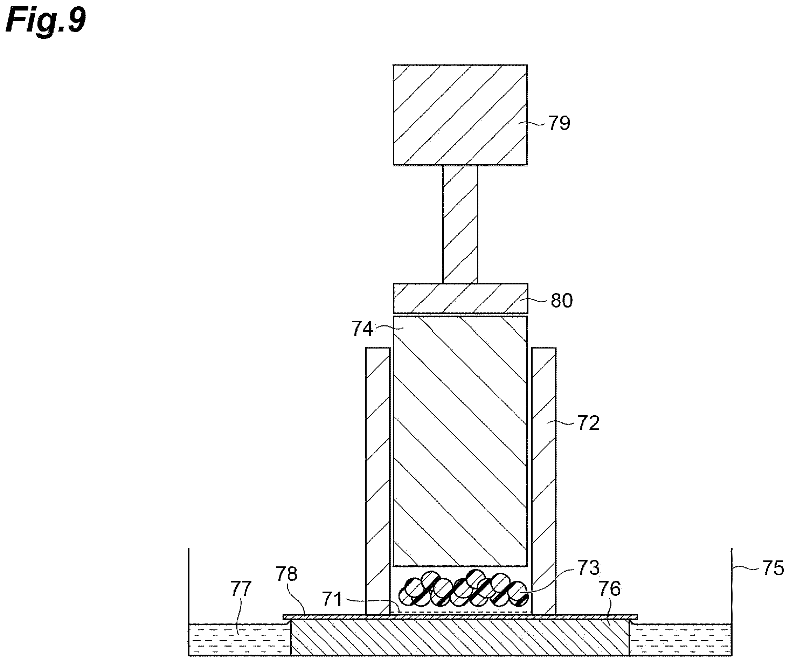

Using the device shown in Fig. 9, a swelling power test of the water-absorbent resin particles A to J and the water absorption layers A and B was performed. The swelling power test was carried out according to the following procedure. A small tabletop tester EZ-Test (Shimadzu Corporation, model: EZ-SX) was used for measuring the swelling power.
(1) An acrylic resin-made cylinder 72 having an inner diameter of 20 mm (bottom area: 3.14 cm²) and a height of 50 mm, with opening portions provided at both ends, in which Nylon (registered trademark) mesh 71 (255 meshes) was mounted in one opening portion, was prepared.
(2) In a state where the cylinder 72 vertically stood in a direction, with the opening portion on the side on which the Nylon (registered trademark) mesh 71 was mounted, facing downward, 0.1 g of water-absorbent resin particles 73 (collected from the water absorption layers A and B in a case of measuring the swelling power of the water absorption layers A and B) were uniformly sprayed on Nylon (registered trademark) mesh 71 of the cylinder 72. The uniformity was visually confirmed. As necessary, the cylinder 72 was shaken to adjust the water-absorbent resin particles 73 to be uniform. An acrylic resin-made cylindrical jig 74 (cylindrical weight) having a diameter of 19.5 mm, a height of 59 mm, and a mass of 20.5 g was inserted into the cylinder 72, and the cylindrical jig 74 was disposed on the water-absorbent resin particles 73.
(3) A petri dish 75 having a diameter of 100 mm was horizontally placed on the measurement table of the EZ-Test. A dried glass filter 76 (filter pore diameter G1) having a diameter of 50 mm and a thickness of 5 mm was placed in the central portion in the above-mentioned Petri dish 75, and physiological saline 77 at 25°C was injected (about 20 ml) to a position slightly below the upper surface of the glass filter 76.
(4) A water impermeable sheet 78 having a size of 50 mm × 50 mm was placed in the vicinity of the center of the upper surface of the glass filter 76 permeated with the physiological saline 77, and the cylinder 72 including the water-absorbent resin particles 73 was vertically placed on the water impermeable sheet 78 in a state where the Nylon (registered trademark) mesh 71 was on the bottom.
(5) A load cell 79 was disposed such that a pressure-sensitive portion having a diameter of 20 mm connected to the load cell 79 of the EZ-Test was positioned immediately above the cylindrical jig 74 in the cylinder 72. More specifically, the measurement table of the EZ-Test was raised and lowered to bring the pressure-sensitive portion connected to the load cell 79 close to the cylindrical jig 74, and the measurement was stopped at a time point when a slight pressure due to the contact was observed. Subsequently, the measurement table of the EZ-Test was moved slightly upward, and a position at which the pressure indication value was 0 ± 0.05 (unit: Newton (N)) was set as a measurement start point.
(6) The water impermeable sheet 78 was promptly removed to allow the water-absorbent resin particles 73 to start water absorption. The time point when the water impermeable sheet 78 was removed was set as an elapsed time t = 0 [sec], a change in time of the force generated by the swelling of the water-absorbent resin particles 73 that absorbed the physiological saline 77 was measured by the load cell 79, and the value at t = 10 [sec] was set as the swelling power at each time. The measurement results of the swelling power are shown in Table 1. In addition, the swelling powers of each of the water absorption layer A and the water absorption layer B of the absorbent articles of Examples and Comparative Examples which will be described later are shown in Table 4.

**[Table 1]**

| Water-absorbent resin particles | Evaluation results of water-absorbent resin particles | | | |
|---|---|---|---|---|
| | Water retention amount (g/g) | Water absorption rate (seconds) | 1-Minute value of lock-up height (cm/g) | 10-Second value of swelling power (N) |
| A | 41 | 35 | 16.0 | 2.0 |
| B | 32 | 44 | 13.5 | 0.5 |
| C | 36 | 36 | 13.0 | 0.1 |
| D | 54 | 41 | 12.5 | 2.2 |
| E | 33 | 29 | 11.0 | 4.6 |
| F | 37 | 46 | 10.0 | 1.4 |
| G | 38 | 307 | 1.0 | 0.0 |
| H | 37 | 447 | 1.0 | 0.0 |
| I | 34 | 782 | 0.5 | 0.0 |
| J | 40 | 583 | 0.5 | 0.0 |

### <Manufacture of Absorber>

### (Production Example A)

An air-laid non-woven fabric (KNH Enterprise Co., Ltd.) having a basis weight of 40 g/m² was cut into two pieces having a size of 42 cm × 14 cm, which were defined as an air-laid non-woven fabric 1 and an air-laid non-woven fabric 2, respectively.

A total amount of 0.2 g of a hot melt adhesive (Henkel Japan Ltd., ME-765E, softening point 96°C) was applied to the air-laid non-woven fabric 2 in 14 straight lines at intervals of 10 mm with a hot melt coating machine (HALLYS Corporation, pump: Marshal 150, table: XA-DT, tank set temperature: 150°C, set temperature in hose: 165°C, gun head set temperature: 170°C). The application pattern of the adhesive was a spiral stripe. During the application, the four sides (width 1 cm) of the outer periphery of the air-laid non-woven fabric 1 were masked so that the adhesive was not applied.

A total of 14.40 g of the water-absorbent resin particles was uniformly sprayed on the central portion (in a range of 40 cm × 12 cm) of the surface of the air-laid non-woven fabric 1 to which the adhesive had adhered.

0.2 g of the hot melt was applied to the air-laid non-woven fabric 2 without masking the outer periphery, the air-laid non-woven fabric 1 and the air-laid non-woven fabric 2 were aligned at both ends so that the adhesive coating surface was on the inside, sandwiched with a release paper, and pressed and bonded using a laminating machine (Hashima Corporation, Straight Linear Fusing Press, model: HP-600LFS) under the conditions of 110°C and 0.1 MPa, and the release paper was peeled off to manufacture an absorber. The range in which the water-absorbent resin particles were sprayed in the absorber was 40 cm × 12 cm. Next, a polyethylene-made air-through type porous liquid permeable sheet having the same area as the upper surface of the absorber and a basis weight of 22 g/m² was disposed on the upper surface of the absorber to manufacture an absorbent article.

### (Production Example B-1)

An absorbent article was produced in the same manner as in Production Example A, except that in the central portion (in a range of 40 cm × 12 cm) of the surface of the air-laid non-woven fabric 1 to which the hot melt was attached, the water absorption layer A (40 cm × 5 cm), the water absorption layer B (40 cm × 2 cm), and the water absorption layer A (40 cm × 5 cm) were provided in this order in the transverse direction of the air-laid non-woven fabric 1, a total of 12.00 g of the water-absorbent resin particles 10a were uniformly sprayed on the two water absorption layers A, and a total of 2.40 g of the water-absorbent resin particles 10b were uniformly sprayed on the water absorption layer B. Furthermore, in the Production Example B-1, the positional relationship between the water absorption layer A and the water absorption layer B in the absorbent article was the same as that in Fig. 2, and the difference (T_{A} - T_{B}) between the thickness (T_{A}) of the water absorption layer A and the thickness (T_{B}) of the water absorption layer B was 0 mm.

### (Production Example B-2)

An absorbent article was produced in the same manner as in Production Example A, except that in the central portion (in a range of 40 cm × 12 cm) of the surface of the air-laid non-woven fabric 1 to which the hot melt was attached, the water absorption layer A (40 cm × 5.5 cm), the water absorption layer B (40 cm × 1 cm), and the water absorption layer A (40 cm × 5.5 cm) were provided in this order in the transverse direction of the air-laid non-woven fabric 1, a total of 13.20 g of the water-absorbent resin particles 10a were uniformly sprayed on the two water absorption layers A, and a total of 1.20 g of the water-absorbent resin particles 10b were uniformly sprayed on the water absorption layer B. Furthermore, in the Production Example B-2, the positional relationship between the water absorption layer A and the water absorption layer B in the absorbent article was the same as that in Fig. 2, and the difference (T_{A} - T_{B}) between the thickness (T_{A}) of the water absorption layer A and the thickness (T_{B}) of the water absorption layer B was 0 mm.

### (Production Example B-3)

An absorbent article was produced in the same manner as in Production Example A, except that in the central portion (in a range of 40 cm × 12 cm) of the surface of the air-laid non-woven fabric 1 to which the hot melt was attached, the water absorption layer A (40 cm × 4.5 cm), the water absorption layer B (40 cm × 3 cm), and the water absorption layer A (40 cm × 4.5 cm) were provided in this order in the transverse direction of the air-laid non-woven fabric 1, a total of 10.80 g of the water-absorbent resin particles 10a were uniformly sprayed on the two water absorption layers A, and a total of 3.60 g of the water-absorbent resin particles 10b were uniformly sprayed on the water absorption layer B. Furthermore, in the Production Example B-3, the positional relationship between the water absorption layer A and the water absorption layer B in the absorbent article was the same as that in Fig. 2, and the difference (T_{A} - T_{B}) between the thickness (T_{A}) of the water absorption layer A and the thickness (T_{B}) of the water absorption layer B was 0 mm.

### (Production Example B-4)

An absorbent article was produced in the same manner as in Production Example A, except that in the central portion (in a range of 40 cm × 12 cm) of the surface of the air-laid non-woven fabric 1 to which the hot melt was attached, the water absorption layer B (20 cm in the longitudinal direction of the air-laid non-woven fabric 1 × 2 cm in the transverse direction of the air-laid non-woven fabric 1), which is line-symmetrical with respect to both the longitudinal centerline and the transverse centerline of the air-laid non-woven fabric 1, the water absorption layer A occupying the remaining area excluding the water absorption layer B were provided, a total of 13.20 g of the water-absorbent resin particles 10a were uniformly sprayed on the water absorption layer A, and a total of 1.20 g of the water-absorbent resin particles 10b were uniformly sprayed on the water absorption layer B. Furthermore, in the Production Example B-4, the positional relationship between the water absorption layer A and the water absorption layer B in the absorbent article was the same as that in Fig. 3, and the difference (T_{A} - T_{B}) between the thickness (T_{A}) of the water absorption layer A and the thickness (T_{B}) of the water absorption layer B was 0 mm.

### (Production Example B-5)

An absorbent article was produced in the same manner as in Production Example A, except that in the central portion (in a range of 40 cm × 12 cm) of the surface of the air-laid non-woven fabric 1 to which the hot melt was attached, the water absorption layer A (40 cm × 4 cm), the water absorption layer B (40 cm × 1 cm), the water absorption layer A (40 cm × 2 cm), the water absorption layer B (40 cm × 1 cm), and the water absorption layer A (40 cm × 4 cm) were provided in this order in the transverse direction of the air-laid non-woven fabric 1, a total of 12.00 g of the water-absorbent resin particles 10a were uniformly sprayed on the three water absorption layers A, and a total of 2.40 g of the water-absorbent resin particles 10b were uniformly sprayed on the two water absorption layers B. Furthermore, in the Production Example B-5, the positional relationship between the water absorption layer A and the water absorption layer B in the absorbent article was the same as that in Fig. 6(a), and the difference (T_{A} - T_{B}) between the thickness (T_{A}) of the water absorption layer A and the thickness (T_{B}) of the water absorption layer B was 0 mm.

### (Production Example C-1)

An absorbent article was produced in the same manner as in Production Example A, except that in the central portion (in a range of 40 cm × 12 cm) of the surface of the air-laid non-woven fabric 1 to which the hot melt was attached, the water absorption layer A (19 cm × 12 cm), the water absorption layer B (2 cm × 12 cm), and the water absorption layer A (19 cm × 12 cm) were provided in this order in the longitudinal direction of the air-laid non-woven fabric 1, a total of 13.68 g of the water-absorbent resin particles 10a were uniformly sprayed on the two water absorption layers A, and a total of 0.72 g of the water-absorbent resin particles 10b were uniformly sprayed on the water absorption layer B. Furthermore, in the Production Example C-1, the positional relationship between the water absorption layer A and the water absorption layer B in the absorbent article was the same as that in Fig. 4, and the difference (T_{A} - T_{B}) between the thickness (T_{A}) of the water absorption layer A and the thickness (T_{B}) of the water absorption layer B was 0 mm.

### (Production Example C-2)

An absorbent article was produced in the same manner as in Production Example A, except that in the central portion (in a range of 40 cm × 12 cm) of the surface of the air-laid non-woven fabric 1 to which the hot melt was attached, the water absorption layer A (19.5 cm × 12 cm), the water absorption layer B (1 cm × 12 cm), and the water absorption layer A (19.5 cm × 12 cm) were provided in this order in the longitudinal direction of the air-laid non-woven fabric 1, a total of 14.04 g of the water-absorbent resin particles 10a were uniformly sprayed on the two water absorption layers A, and a total of 0.36 g of the water-absorbent resin particles 10b were uniformly sprayed on the water absorption layer B. Furthermore, in Production Example C-2, the positional relationship between the water absorption layer A and the water absorption layer B in the absorbent article was the same as that in Fig. 4, and the difference (T_{A} - T_{B}) between the thickness (T_{A}) of the water absorption layer A and the thickness (T_{B}) of the water absorption layer B was 0 mm.

### (Production Example C-3)

An absorbent article was produced in the same manner as in Production Example A, except that in the central portion (in a range of 40 cm × 12 cm) of the surface of the air-laid non-woven fabric 1 to which the hot melt was attached, the water absorption layer A (18.5 cm × 12 cm), the water absorption layer B (3 cm × 12 cm), and the water absorption layer A (18.5 cm × 12 cm) were provided in this order in the longitudinal direction of the air-laid non-woven fabric 1, a total of 13.32 g of the water-absorbent resin particles 10a were uniformly sprayed on the two water absorption layers A, and a total of 1.08 g of the water-absorbent resin particles 10b were uniformly sprayed on the water absorption layer B. Furthermore, in Production Example C-3, the positional relationship between the water absorption layer A and the water absorption layer B in the absorbent article was the same as that in Fig. 4, and the difference (T_{A} - T_{B}) between the thickness (T_{A}) of the water absorption layer A and the thickness (T_{B}) of the water absorption layer B was 0 mm.

### (Production Example C-4)

An absorbent article was produced in the same manner as in Production Example A, except that in the central portion (in a range of 40 cm × 12 cm) of the surface of the air-laid non-woven fabric 1 to which the hot melt was attached, the water absorption layer B (2 cm in the longitudinal direction of the air-laid non-woven fabric 1 × 8 cm in the transverse direction of the air-laid non-woven fabric 1), which is line-symmetrical with respect to both the longitudinal centerline and the transverse centerline of the air-laid non-woven fabric 1, and the water absorption layer A occupying the remaining area excluding the water absorption layer B were provided. A total of 13.92 g of the water-absorbent resin particles 10a were uniformly sprayed on the water absorption layer A, and a total of 0.48 g of the water-absorbent resin particles 10b were uniformly sprayed on the water absorption layer B. Furthermore, in Production Example C-4, the positional relationship between the water absorption layer A and the water absorption layer B in the absorbent article was the same as that in Fig. 5, and the difference (T_{A} - T_{B}) between the thickness (T_{A}) of the water absorption layer A and the thickness (T_{B}) of the water absorption layer B was 0 mm.

### <Production of Absorbent Articles of Examples and Comparative Examples>

### (Comparative Example 1)

In Production Example A, an absorbent article was produced using 14.40 g of the water-absorbent resin particles E obtained in Production Example 6.

### (Comparative Example 2)

In Production Example A, an absorbent article was produced using 14.40 g of the water-absorbent resin particles C obtained in Production Example 4.

### (Comparative Example 3)

In Production Example A, an absorbent article was produced using 14.40 g of the water-absorbent resin particles A obtained in Production Example 2.

### (Comparative Example 4)

In Production Example A, an absorbent article was produced using 14.40 g of the water-absorbent resin particles D obtained in Production Example 5.

### (Example 1)

In Production Example B-1, an absorbent article was manufactured using 12.00 g of the water-absorbent resin particles E obtained in Production Example 6 in the water absorption layer A, and a mixture of 0.96 g of the water-absorbent resin particles A obtained in Production Example 2 and 1.44 g of the water-absorbent resin particles H obtained in Production Example 10 in the water absorption layer B. The difference (W_{A} - W_{B}) between the mass (W_{A}) of the water-absorbent resin particles per unit area of the water absorption layer A and the mass (W_{B}) of the water-absorbent resin particles per unit area of the water absorption layer B was 0 g/cm², and the difference (T_{A} - T_{B}) between the thickness (T_{A}) of the water absorption layer A and the thickness (T_{B}) of the water absorption layer B was 0 mm.

### (Example 2)

In Production Example B-1, an absorbent article was manufactured using 12.00 g of the water-absorbent resin particles C obtained in Production Example 4 in the water absorption layer A, and a mixture of 0.96 g of the water-absorbent resin particles A obtained in Production Example 2 and 1.44 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 3)

In Production Example B-1, an absorbent article was manufactured using 12.00 g of the water-absorbent resin particles A obtained in Production Example 2 in the water absorption layer A, and a mixture of 0.96 g of the water-absorbent resin particles A obtained in Production Example 2 and 1.44 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 4)

In Production Example B-1, an absorbent article was manufactured using 12.00 g of the water-absorbent resin particles D obtained in Production Example 5 in the water absorption layer A, and a mixture of 0.96 g of the water-absorbent resin particles A obtained in Production Example 2 and 1.44 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 5)

In Production Example B-1, an absorbent article was manufactured using 12.00 g of the water-absorbent resin particles A obtained in Production Example 2 in the water absorption layer A, and a mixture of 0.96 g of the water-absorbent resin particles E obtained in Production Example 6 and 1.44 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 6)

In Production Example B-1, an absorbent article was manufactured using 12.00 g of the water-absorbent resin particles A obtained in Production Example 2 in the water absorption layer A, and a mixture of 0.96 g of the water-absorbent resin particles C obtained in Production Example 4 and 1.44 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 7)

In Production Example B-1, an absorbent article was manufactured using a mixture of 12.00 g of the water-absorbent resin particles A obtained in Production Example 2 in the water absorption layer A, and a mixture of 0.96 g of the water-absorbent resin particles D obtained in Production Example 5 and 1.44 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 8)

In Production Example B-1, an absorbent article was manufactured using 12.00 g of the water-absorbent resin particles A obtained in Production Example 2 in the water absorption layer A, and a mixture of 0.96 g of the water-absorbent resin particles A obtained in Production Example 2 and 1.44 g of the water-absorbent resin particles G obtained in Production Example 9 in the water absorption layer B. The difference (W_{A} - W_{B}) was 0 g/cm², and the difference (T_{A} - T_{B}) was 0 mm.

### (Example 9)

In Production Example B-1, an absorbent article was manufactured using 12.00 g of the water-absorbent resin particles A obtained in Production Example 2 in the water absorption layer A, and a mixture of 0.96 g of the water-absorbent resin particles A obtained in Production Example 2 and 1.44 g of the water-absorbent resin particles I obtained in Production Example 11 in the water absorption layer B. The difference (W_{A} - W_{B}) was 0 g/cm², and the difference (T_{A} - T_{B}) was 0 mm.

### (Example 10)

In Production Example B-1, an absorbent article was manufactured using 12.00 g of the water-absorbent resin particles A obtained in Production Example 2 in the water absorption layer A, and a mixture of 0.96 g of the water-absorbent resin particles A obtained in Production Example 2 and 1.44 g of the water-absorbent resin particles J obtained in Production Example 13 in the water absorption layer B. The difference (W_{A} - W_{B}) was 0 g/cm², and the difference (T_{A} - T_{B}) was 0 mm.

### (Example 11)

In Production Example B-1, an absorbent article was manufactured using 12.00 g of the water-absorbent resin particles A obtained in Production Example 2 in the water absorption layer A, and a mixture of 0.96 g of the water-absorbent resin particles A obtained in Production Example 2 and 1.44 g of the water-absorbent resin particles B obtained in Production Example 3 in the water absorption layer B. The difference (W_{A} - W_{B}) was 0 g/cm², and the difference (T_{A}- T_{B}) was 0 mm.

### (Example 12)

In Production Example B-1, an absorbent article was manufactured using 12.00 g of the water-absorbent resin particles A obtained in Production Example 2 in the water absorption layer A, and a mixture of 0.96 g of the water-absorbent resin particles A obtained in Production Example 2 and 1.44 g of the water-absorbent resin particles C obtained in Production Example 4 in the water absorption layer B. The difference (W_{A} - W_{B}) was 0 g/cm², and the difference (T_{A} - T_{B}) was 0 mm.

### (Example 13)

In Production Example B-1, an absorbent article was manufactured using 12.00 g of the water-absorbent resin particles A obtained in Production Example 2 in the water absorption layer A, and a mixture of 0.96 g of the water-absorbent resin particles A obtained in Production Example 2 and 1.44 g of the water-absorbent resin particles E obtained in Production Example 6 in the water absorption layer B. The difference (W_{A} - W_{B}) was 0 g/cm², and the difference (T_{A} - T_{B}) was 0 mm.

### (Example 14)

In Production Example B-1, an absorbent article was manufactured using 12.00 g of the water-absorbent resin particles A obtained in Production Example 2 in the water absorption layer A, and a mixture of 0.96 g of the water-absorbent resin particles A obtained in Production Example 2 and 1.44 g of the water-absorbent resin particles F obtained in Production Example 7 in the water absorption layer B. The difference (W_{A} - W_{B}) was 0 g/cm², and the difference (T_{A} - T_{B}) was 0 mm.

### (Example 15)

In Production Example B-2, an absorbent article was manufactured using 13.20 g of the water-absorbent resin particles A obtained in Production Example 2 in the water absorption layer A, and a mixture of 0.48 g of the water-absorbent resin particles A obtained in Production Example 2 and 0.72 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 16)

In Production Example B-3, an absorbent article was manufactured using 10.80 g of the water-absorbent resin particles A obtained in Production Example 2 in the water absorption layer A, and a mixture of 1.44 g of the water-absorbent resin particles A obtained in Production Example 2 and 2.16 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 17)

In Production Example B-4, an absorbent article was manufactured using 13.20 g of the water-absorbent resin particles A obtained in Production Example 2 in the water absorption layer A, and a mixture of 0.48 g of the water-absorbent resin particles A obtained in Production Example 2 and 0.72 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 18)

In Production Example B-5, an absorbent article was manufactured using 12.00 g of the water-absorbent resin particles A obtained in Production Example 2 in the water absorption layer A, and a mixture of 0.96 g of the water-absorbent resin particles A obtained in Production Example 2 and 1.44 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 19)

In Production Example C-2, an absorbent article was manufactured using 14.04 g of the water-absorbent resin particles A obtained in Production Example 2 in the water absorption layer A, and a mixture of 0.14 g of the water-absorbent resin particles A obtained in Production Example 2 and 0.22 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 20)

In Production Example C-1, an absorbent article was manufactured using 13.68 g of the water-absorbent resin particles A obtained in Production Example 2 in the water absorption layer A, and a mixture of 0.29 g of the water-absorbent resin particles A obtained in Production Example 2 and 0.43 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 21)

In Production Example C-3, an absorbent article was manufactured using 13.32 g of the water-absorbent resin particles A obtained in Production Example 2 in the water absorption layer A, and a mixture of 0.43 g of the water-absorbent resin particles A obtained in Production Example 2 and 0.65 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 22)

In Production Example C-4, an absorbent article was manufactured using 13.92 g of the water-absorbent resin particles A obtained in Production Example 2 in the water absorption layer A, and a mixture of 0.19 g of the water-absorbent resin particles A obtained in Production Example 2 and 0.29 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 23)

In Production Example B-1, an absorbent article was manufactured using 12.00 g of the water-absorbent resin particles A obtained in Production Example 2 in the water absorption layer A, and a mixture of 1.92 g of the water-absorbent resin particles A obtained in Production Example 2 and 0.48 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 24)

In Production Example B-1, an absorbent article was manufactured using 12.00 g of the water-absorbent resin particles A obtained in Production Example 2 in the water absorption layer A, and a mixture of 0.72 g of the water-absorbent resin particles A obtained in Production Example 2 and 1.68 g of the water-absorbent resin particles H obtained in Production Example 10 in the water absorption layer B. The difference (W_{A} - W_{B}) was 0 g/cm², and the difference (T_{A} - T_{B}) was 0 mm.

### (Example 25)

In Production Example B-1, an absorbent article was manufactured using 12.00 g of the water-absorbent resin particles A obtained in Production Example 2 in the water absorption layer A, and a mixture of 0.24 g of the water-absorbent resin particles A obtained in Production Example 2 and 2.16 g of the water-absorbent resin particles H obtained in Production Example 10 in the water absorption layer B. The difference (W_{A} - W_{B}) was 0 g/cm², and the difference (T_{A} - T_{B}) was 0 mm.

### (Comparative Example 5)

In Production Example B-1, an absorbent article was manufactured using 12.00 g of the water-absorbent resin particles A obtained in Production Example 2 in the water absorption layer A, and 0.96 g of the water-absorbent resin particles A obtained in Production Example 2 in the water absorption layer B. The difference (W_{A} - W_{B}) was 0.018 g/cm², and the difference (T_{A} - T_{B}) was 0.5 mm.

### <Evaluation of Absorbent Article>

### [Preparationof Test Solution (Artificial Urine)]

9,866.0 g of distilled water, 100.0 g of sodium chloride, 3.0 g of calcium chloride dihydrate, 6.0 g of magnesium chloride hexahydrate, 25.0 g of a 1% by mass Triton X solution (mixture of Triton X-100 manufactured by FUJIFILM Wako Pure Chemical Corporation and water), and 0.25 g of food blue No. 1 (for coloration) were mixed to prepare a test solution.

### [Permeation Rate]

In a room adjusted to 25°C and a humidity (RH) of 50%, the permeation rate was measured by the following procedure. An absorbent article was placed on a horizontal table, a liquid injection cylinder (59.80 g) having an opening portion with an outer diameter of 3.5 cm and an inner diameter of 3 cm was placed on the central portion of the absorbent article, and 80 mL of the test solution was injected into the cylinder at one time. The time from the time point when the injection of the test solution was started to the time pointed when the test solution was completely absorbed by the absorbent article was measured as the permeation rate (first round). The liquid injection cylinder was removed from the absorbent article immediately after the test solution was completely absorbed by the absorbent article.

Next, 30 minutes after the time point when the injection of the test solution was started, the permeation rate (second round) was measured according to the same procedure as that for the first round permeation rate.

Next, 30 minutes after the time point when the second round injection of the test solution was started, the permeation rate (third round) was measured according to the same procedure as that for the third round permeation rate. The (total) permeation rate was calculated by summing the permeation rates from all three rounds. The measurement results are shown in Tables 2 and 3.

### [45-Degree Leakage Test]

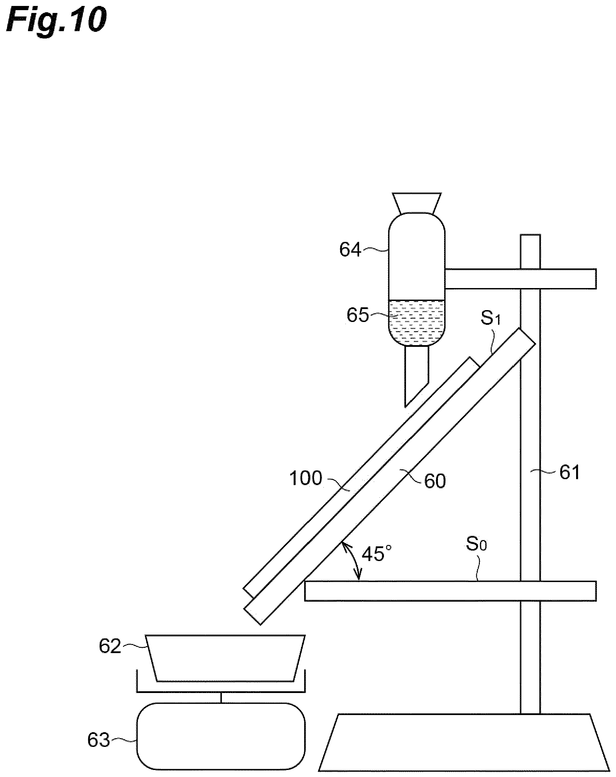

Fig. 10 is a schematic view showing a method for evaluating leakage properties of the absorbent article. A support plate 60 (acrylic resin plate) having a length of 45 cm and a thickness of 0.3 cm and having a flat inclined surface was fixed by a mount 61 in a state of being inclined by 45° ± 2° with respect to the horizontal plane S0. A dropping funnel with a volume of 300 mL had a tip end part with an inner diameter of about 8 mmφ and a two-way cock, and a stop of the cock was adjusted such that a liquid was injected at 8 mL/sec. A balance 63 on which a metal tray 62 was placed was installed at the lower part of the support plate 60 to receive all drops of the test solution flowing down as leaks from the end of the absorbent article, and a mass thereof was recorded with an accuracy of 0.1 g.

A 45-degree leakage test was performed by the following procedure in a room adjusted to 25°C and a humidity of 50% (RH). An absorbent article 100 for testing was attached onto an inclined surface S1 of a fixed support plate 60 such that the longitudinal direction of the absorbent article 100 was along the longitudinal direction of the support plate 60. The lower end of the absorbent article was not attached onto the acrylic plate to avoid intentionally stopping the leakage. Next, 80 mL of a test solution 65 (artificial urine) adjusted to 25°C ± 1°C was added dropwise from a dropping funnel 64 disposed vertically above the absorbent article to a position 100 mm from the center to the upper end of the absorber in the absorbent article 100. A distance between the tip end of the dropping funnel 64 and the absorbent article was 10 ± 2 mm. The surface of the support plate 60 was smooth, and the liquid did not stay on or was not absorbed by the plate.

In a case where the test solution not absorbed by the absorbent article 100 leaked out from the lower part of the support plate 60, the leaked test solution was recovered in the metal tray 62 disposed below the support plate 60. A weight (g) of the recovered test solution was measured by a balance 63 and this value was recorded as an amount of leakage. Next, 10 minutes after the time point when the injection of the test solution was started, a second round injection of the test solution was performed in the same manner as in the first round addition, and these operations were repeated for 5 additional rounds for a total of 7 rounds. The total amount of the test solution absorbed by the absorber at a time point when the test solution was completely injected over seven rounds was calculated from the following expression and evaluated as the absorption capacity of the absorbent article 100. The measurement results of the absorption capacity are shown in Tables 2 and 3. Absorption capacity [g] = 80 [ml] × 7 [rounds] - Total leakage amount [g] up to seventh round

**[Table 2]**

| | Area ratio of water absorption layer B | Water-absorbent resin particles | | | | | | Thickness (mm) | Evaluation results of absorbent article | | | | |
|---|---|---|---|---|---|---|---|---|---|---|---|---|---|
| | | Water absorption layer A | | Water absorption layer B | | | | | Permeation rate (seconds) | | | | Absorption capacity (g) |
| | | Type | Mass (g) | Type | Mass (g) | Type | Mass (g) | | First round | Second round | Third round | Total | |
| Comparative Example 1 | 0.0% | E | 14.40 | - | - | - | - | 2.1 | 36 | 16 | 18 | 70 | 375 |
| Comparative Example 2 | | C | 14.40 | - | - | - | - | 2.1 | 37 | 18 | 24 | 79 | 478 |
| Comparative Example 3 | | A | 14.40 | - | - | - | - | 2.1 | 42 | 30 | 35 | 107 | 520 |
| Comparative Example 4 | | D | 14.40 | - | - | - | - | 2.2 | 41 | 40 | 41 | 122 | 548 |
| Example 1 | 16.7% | E | 12.00 | A | 0.96 | H | 1.44 | 2.3 | 32 | 16 | 17 | 65 | 397 |
| Example 2 | | C | 12.00 | A | 0.96 | H | 1.44 | 2.1 | 38 | 14 | 18 | 70 | 461 |
| Example 3 | | A | 12.00 | A | 0.96 | H | 1.44 | 2.1 | 36 | 17 | 20 | 73 | 491 |
| Example 4 | | D | 12.00 | A | 0.96 | H | 1.44 | 2.1 | 39 | 24 | 29 | 92 | 513 |
| Example 5 | | A | 12.00 | E | 0.96 | H | 1.44 | 2.1 | 36 | 16 | 17 | 69 | 482 |
| Example 6 | | A | 12.00 | C | 0.96 | H | 1.44 | 2.1 | 34 | 18 | 16 | 68 | 487 |
| Example 7 | | A | 12.00 | D | 0.96 | H | 1.44 | 2.1 | 36 | 15 | 25 | 76 | 509 |
| Example 8 | | A | 12.00 | A | 0.96 | G | 1.44 | 2.0 | 38 | 16 | 24 | 78 | 504 |
| Example 9 | | A | 12.00 | A | 0.96 | I | 1.44 | 2.1 | 32 | 17 | 19 | 68 | 473 |
| Example 10 | | A | 12.00 | A | 0.96 | J | 1.44 | 2.0 | 38 | 15 | 20 | 73 | 493 |
| Example 11 | | A | 12.00 | A | 0.96 | B | 1.44 | 2.1 | 31 | 23 | 25 | 79 | 501 |
| Example 12 | | A | 12.00 | A | 0.96 | C | 1.44 | 2.1 | 34 | 26 | 31 | 91 | 519 |
| Example 13 | | A | 12.00 | A | 0.96 | E | 1.44 | 2.1 | 33 | 19 | 21 | 73 | 481 |
| Example 14 | | A | 12.00 | A | 0.96 | F | 1.44 | 2.1 | 32 | 24 | 24 | 80 | 500 |

**[Table 3]**

| | Area ratio of water absorption layer B | Water-absorbent resin particles | | | | | | Thickness (mm) | Evaluation results of absorbent article | | | | |
|---|---|---|---|---|---|---|---|---|---|---|---|---|---|
| | | Water absorption layer A | | Water absorption layer B | | | | | Permeation rate (seconds) | | | | Absorption capacity (g) |
| | | Type | Mass (g) | Type | Mass (g) | Type | Mass (g) | | First round | Second round | Third round | Total | |
| Example 15 | 8.3% | A | 13.20 | A | 0.48 | H | 0.72 | 2.1 | 36 | 20 | 20 | 76 | 512 |
| Example 16 | 25.0% | A | 10.80 | A | 1.44 | H | 2.16 | 2.1 | 38 | 16 | 20 | 74 | 469 |
| Example 17 | 8.3% | A | 13.20 | A | 0.48 | H | 0.72 | 2.1 | 36 | 18 | 20 | 74 | 507 |
| Example 18 | 16.7% | A | 12.00 | A | 0.96 | H | 1.44 | 2.1 | 35 | 23 | 21 | 79 | 497 |
| Example 19 | 2.5% | A | 14.04 | A | 0.14 | H | 0.22 | 2.1 | 29 | 19 | 22 | 70 | 512 |
| Example 20 | 5.0% | A | 13.68 | A | 0.29 | H | 0.43 | 2.1 | 31 | 11 | 16 | 58 | 510 |
| Example 21 | 7.5% | A | 13.32 | A | 0.43 | H | 0.65 | 2.1 | 32 | 10 | 16 | 58 | 504 |
| Example 22 | 3.3% | A | 13.92 | A | 0.19 | H | 0.29 | 2.1 | 35 | 15 | 22 | 72 | 499 |
| Example 23 | 16.7% | A | 12.00 | A | 1.92 | H | 0.48 | 2.1 | 35 | 27 | 26 | 88 | 506 |
| Example 24 | | A | 12.00 | A | 0.72 | H | 1.68 | 2.1 | 33 | 13 | 16 | 62 | 478 |
| Example 25 | | A | 12.00 | A | 0.24 | H | 2.16 | 2.1 | 35 | 6 | 7 | 48 | 463 |
| Comparative Example 5 | 16.7% | A | 12.00 | A | 0.96 | - | - | Water absorption layer A: 2.1 | 37 | 15 | 17 | 69 | 421 |
| | | | | | | | | Water absorption layer B: 1.6 | | | | | |

**[Table 4]**

| | 1-Minute value of lock-up height (cm/g) | | | 10-Second value of swelling power (N) | | Total permeation rate (seconds) | Absorption capacity (g) |
|---|---|---|---|---|---|---|---|
| | Water absorption layer A | Water absorption layer B | Water absorption layer A - Water absorption layer B | Water absorption layer A | Water absorption layer B | | |
| | 1-Minute value | 1-Minute value | 1-Minute value | 10-Second value | 10-Second value | | |
| Comparative Example 1 | 11.0 | 11.0 | 0 | 4.6 | 4.6 | 70 | 375 |
| Comparative Example 2 | 13.0 | 13.0 | 0 | 0.1 | 0.1 | 79 | 478 |
| Comparative Example 3 | 16.0 | 16.0 | 0 | 2.0 | 2.0 | 107 | 520 |
| Comparative Example 4 | 12.5 | 12.5 | 0 | 2.2 | 2.2 | 122 | 548 |
| Example 1 | 11.0 | 7.0 | 4.0 | 4.6 | 0.8 | 65 | 397 |
| Example 2 | 13.0 | 7.0 | 6.0 | 0.1 | 0.8 | 70 | 461 |
| Example 3 | 16.0 | 7.0 | 9.0 | 2.0 | 0.8 | 73 | 491 |
| Example 4 | 12.5 | 7.0 | 5.5 | 2.2 | 0.8 | 92 | 513 |
| Example 5 | 16.0 | 5.0 | 11.0 | 2.0 | 1.8 | 69 | 482 |
| Example 6 | 16.0 | 5.8 | 10.2 | 2.0 | 0.0 | 68 | 487 |
| Example 7 | 16.0 | 5.6 | 10.4 | 2.0 | 0.9 | 76 | 509 |
| Example 8 | 16.0 | 7.0 | 9.0 | 2.0 | 0.8 | 78 | 504 |
| Example 9 | 16.0 | 6.7 | 9.3 | 2.0 | 0.8 | 68 | 473 |
| Example 10 | 16.0 | 6.7 | 9.3 | 2.0 | 0.8 | 73 | 493 |
| Example 11 | 16.0 | 14.5 | 1.5 | 2.0 | 1.1 | 79 | 501 |
| Example 12 | 16.0 | 14.2 | 1.8 | 2.0 | 0.8 | 91 | 519 |
| Example 13 | 16.0 | 13.0 | 3.0 | 2.0 | 3.6 | 73 | 481 |
| Example 14 | 16.0 | 12.4 | 3.6 | 2.0 | 1.6 | 80 | 500 |
| Example 15 | 16.0 | 7.0 | 9.0 | 2.0 | 0.8 | 76 | 512 |
| Example 16 | 16.0 | 7.0 | 9.0 | 2.0 | 0.8 | 74 | 469 |
| Example 17 | 16.0 | 7.0 | 9.0 | 2.0 | 0.8 | 74 | 507 |
| Example 18 | 16.0 | 7.0 | 9.0 | 2.0 | 0.8 | 79 | 497 |
| Example 19 | 16.0 | 7.0 | 9.0 | 2.0 | 0.8 | 70 | 512 |
| Example 20 | 16.0 | 7.0 | 9.0 | 2.0 | 0.8 | 58 | 510 |
| Example 21 | 16.0 | 7.0 | 9.0 | 2.0 | 0.8 | 58 | 504 |
| Example 22 | 16.0 | 7.0 | 9.0 | 2.0 | 0.8 | 72 | 499 |
| Example 23 | 16.0 | 13.0 | 3.0 | 2.0 | 1.6 | 88 | 506 |
| Example 24 | 16.0 | 5.5 | 10.5 | 2.0 | 0.6 | 62 | 478 |
| Example 25 | 16.0 | 2.5 | 13.5 | 2.0 | 0.2 | 48 | 463 |
| Comparative Example 5 | 16.0 | 6.4 | 9.6 | 2.0 | 2.0 | 69 | 421 |

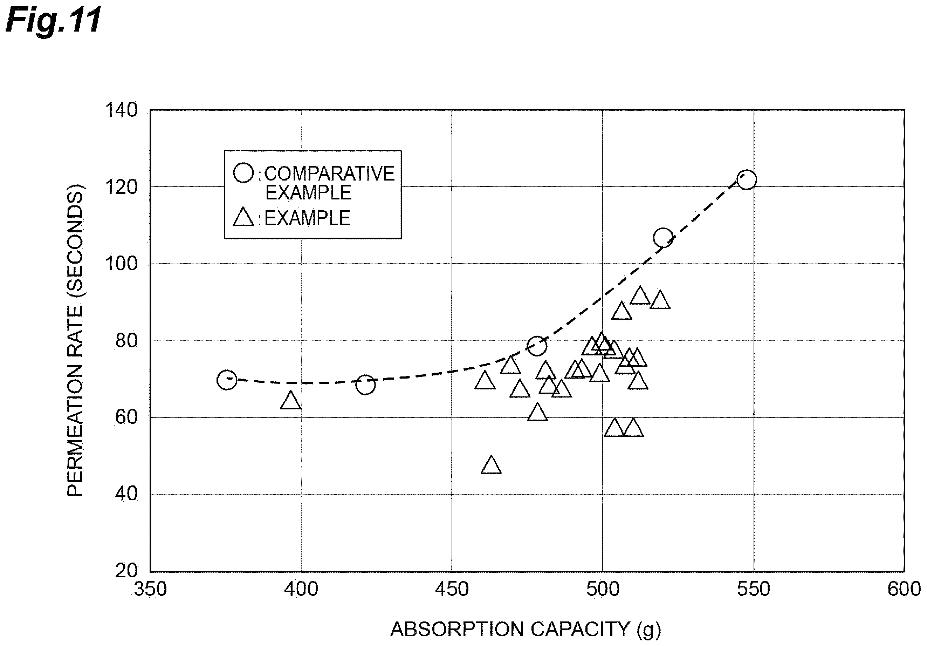

In a case of comparing Comparative Example 1 with Examples 1 to 25, it can be seen that in any of Examples, the absorption capacity can be increased and the absorbent article has a large absorption capacity. In addition, in a case of comparing Comparative Example 4 with Examples 1 to 25, it can be seen that in any of Examples, the permeation rate can be increased and the absorbent article has a high permeation rate. Moreover, in a case of comparing Comparative Example 1 with Example 1 having a similar absorption capacity to that in Comparative Example 1, it can be seen that in Example 1, the permeation rate can be increased while maintaining the absorption capacity, and the absorbent article has a high permeation rate and a large absorption capacity. Furthermore, in a case of comparing Comparative Example 2 with Examples 2, 3, 5, 6, 9, 10, 13, 16, 18, 22, 24, and 25 having an absorption capacity similar to that in Comparative Example 2, it can be seen that in any of Examples, the permeation rate can be increased or maintained while increasing or maintaining the absorption capacity, and the absorbent article has a high permeation rate and a large absorption capacity. In addition, in a case of comparing Comparative Example 3 with Examples 4, 7, 8, 11, 12, 14, 15, 17, 19 to 21, and 23 having an absorption capacity similar to that in Comparative Example 3, it can be seen that in any of Examples, the permeation rate can be increased or maintained while increasing or maintaining the absorption capacity, and the absorbent article has a high permeation rate and a large absorption capacity. These facts can also be confirmed from the graph shown in Fig. 11. Specifically, it can be seen that in any of Examples, the absorption capacity is increased below the wavy line connecting Comparative Examples, the permeation rate can be increased with the same absorption capacity, and an absorbent article having a high permeation rate and a large absorption capacity is obtained.

### Reference Signs List

1, 2, 3, A, B: water absorption layer
10a, 10b: water-absorbent resin particles
11a, 11b: fiber layer
20a, 20b: core wrap sheet
21: adhesive layer
30: liquid permeable sheet
40: liquid impermeable sheet
50: absorber
100: absorbent article

## Claims

1. An absorber comprising:
a water absorption layer comprising water-absorbent resin particles,
wherein the water absorption layer has a water absorption layer A and a water absorption layer B,
the water absorption layer B has a strip shape and the water absorption layers A are disposed on both sides of the water absorption layer B in a transverse direction,
the water absorption layer A has a 10-second value of swelling power of less than 5 N, and
in a case where a 1-minute value of a lock-up height of the water absorption layer A is H_{A} and a 1-minute value of a lock-up height of the water absorption layer B is H_{B}, H_{A} - H_{B} is 1.5 cm/g or more.

2. The absorber according to Claim 1,
wherein an area of the water absorption layer B is 30% or less with respect to a total area of the water absorption layer.

3. The absorber according to Claim 1 or 2,
wherein an area of the water absorption layer B is less than 20% of a total area of the water absorption layer.

4. The absorber according to Claim 1 or 2,
wherein the absorber has a strip shape, and
the water absorption layer B is disposed such that a longitudinal direction of the water absorption layer B is along a longitudinal direction of the absorber.

5. The absorber according to Claim 1 or 2,
wherein the water absorption layer comprises a plurality of the water absorption layers B.

6. Water-absorbent resin particles comprising:
water-absorbent resin particles x having a 1-minute value of a lock-up height of less than 9 cm/g; and
water-absorbent resin particles y having a 1-minute value of a lock-up height of 9 cm/g or more.

7. The water-absorbent resin particles according to Claim 6,
wherein the water-absorbent resin particles x comprises polymer particles and a coating layer covering at least a part of a surface of the polymer particles.

8. An absorber comprising:
the water-absorbent resin particles according to Claim 6.

9. An absorbent article comprising:
the absorber according to Claim 1, 2, or 8.
